# EUROPEAN PATENT APPLICATION

(11) **EP 2 230 251 A1**
(43) Date of publication of application: **22.09.2010**
(21) Application number: 09155614.2
(22) Date of filing: 19.03.2009
(51) Int. Cl.: C07K 16/18

(54) **Antibodies specifically active in the coronary plaque and method for their identification**

(71) Applicant: Bracco Imaging S.p.A, 20134 Milano (IT)
(72) Inventor: Clementi, Massimo, 20132 Milano (IT); Burioni, Roberto, 20132 Milano (IT)
(74) Representative: Macchetta, Francesco

(57) **Abstract**

A process is provided for the preparation of antibodies or fragments thereof using a prokaryotic host cell containing DNA sequences encoding for said antibodies or fragments thereof, wherein said DNA sequence is derived from a coronary plaque sample.
Compositions containing said antibodies are also provided.
Ligands to said antibodies and compositions containing said ligands are also described.

SEQUENCE LISTING

<110>  Bracco Imaging Spa

<120>  New antibodies specifically active in coronary plaque and method
       for their identification

<130>  B0640

<160>  8

<170>  PatentIn version 3.3

<210>  1
<211>  339
<212>  DNA
<213>  Homo sapiens

<400>  1
ctcgaggagt ctggagcaga agtgaagaag ccgggcgaaa atcttaagat ctcctgcgag      60

gcttctggat acaattttgt caatcactgg atcggctggg tgcgccagat gcccgggaga     120

ggccttgagt ggatgggccg catctatcct ggagactctg aaaccagatt cagtccgtcc     180

ttccaagggc aggtcaccat ctcagtcgac aaaactctga gtaccgcctc cctacagtgg     240

aacagtctca agacgtcgga cagcgccaca tattattgtg tgacactggg gcgctggagc     300

agctggcaag gtggggcgct ctcatggggc cagggaacc                            339

<210>  2
<211>  113
<212>  PRT
<213>  Homo sapiens

<400>  2

Leu Glu Glu Ser Gly Ala Glu Val Lys Lys Pro Gly Glu Asn Leu Lys
1               5                   10                  15

Ile Ser Cys Glu Ala Ser Gly Tyr Asn Phe Val Asn His Trp Ile Gly
            20                  25                  30

Trp Val Arg Gln Met Pro Gly Arg Gly Leu Glu Trp Met Gly Arg Ile
        35                  40                  45

Tyr Pro Gly Asp Ser Glu Thr Arg Phe Ser Pro Ser Phe Gln Gly Gln
    50                  55                  60

Val Thr Ile Ser Val Asp Lys Thr Leu Ser Thr Ala Ser Leu Gln Trp
65                  70                  75                  80

Asn Ser Leu Lys Thr Ser Asp Ser Ala Thr Tyr Tyr Cys Val Thr Leu
                85                  90                  95

Gly Arg Trp Ser Ser Trp Gln Gly Gly Ala Leu Ser Trp Gly Gln Gly
            100                 105                 110

Thr

<210>  3
<211>  303
<212>  DNA
<213>  Homo sapiens

<400>  3
gagctcacgc agtctccagc caccgtgtct gtgtctccag gggaaagagc caccctctcc      60

tgcagggcca gtcagagtat tagtttccac ttagcctggt accagcagaa acctggccag     120

gctcccagtc tcctcatcta cggaacatcc accagggcca ctggtatccc agccaggttc     180

agtggcagtg ggtctgggac agagttcact ctcaccatca gcagcctgca gtctgaagat     240

tctgcggttt attactgtca gcagtatcat aactggcctc ccctcacttt cggcggaggg     300

acc                                                                   303

<210>  4
<211>  101
<212>  PRT
<213>  Homo sapiens

<400>  4

Glu Leu Thr Gln Ser Pro Ala Thr Val Ser Val Ser Pro Gly Glu Arg
1               5                   10                  15

Ala Thr Leu Ser Cys Arg Ala Ser Gln Ser Ile Ser Phe His Leu Ala
            20                  25                  30

Trp Tyr Gln Gln Lys Pro Gly Gln Ala Pro Ser Leu Leu Ile Tyr Gly
        35                  40                  45

Thr Ser Thr Arg Ala Thr Gly Ile Pro Ala Arg Phe Ser Gly Ser Gly
    50                  55                  60

Ser Gly Thr Glu Phe Thr Leu Thr Ile Ser Ser Leu Gln Ser Glu Asp
65                  70                  75                  80

Ser Ala Val Tyr Tyr Cys Gln Gln Tyr His Asn Trp Pro Pro Leu Thr
                85                  90                  95

Phe Gly Gly Gly Thr
            100

<210>  5
<211>  12
<212>  PRT
<213>  Homo sapiens

<400>  5

Thr Met Gly Phe Thr Ala Pro Arg Phe Pro His Tyr
1               5                   10

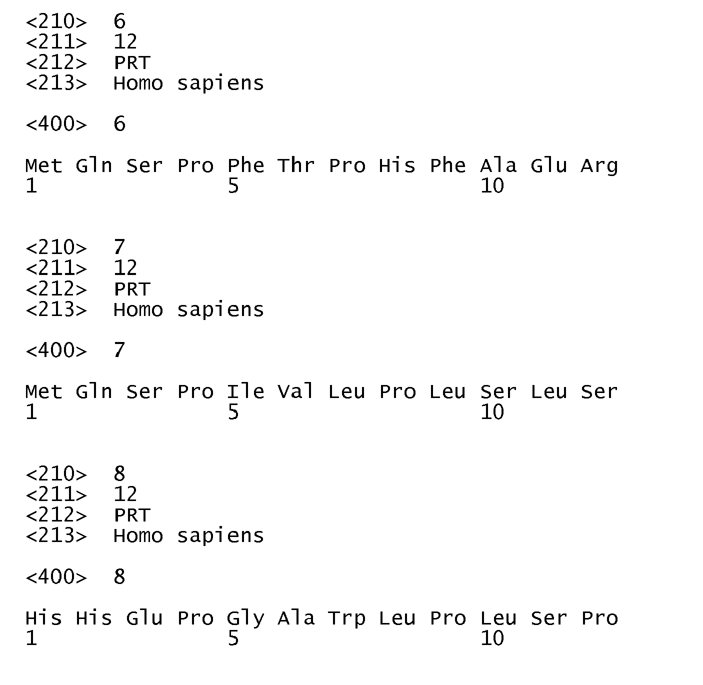

## Description

The present invention relates to a method for preparing new oligoclonal antibodies, the antibodies themselves as well as fragments thereof and their uses as well as the antigen and ligands thereof. In particular, the present invention encompasses antibodies or fragments thereof that are directed against antigens possibly found in the coronary plaque. The present invention further relates to the nucleotidic sequences coding for these antibodies and amino acidic sequences of the antibodies or fragments thereof for use in immunoassays, as well as to the ligands of these antibodies or fragments thereof. Further, the invention encompasses diagnostic and therapeutic applications related to the use of said antibodies or fragments thereof or of their ligands.

### Background

The acute coronary syndrome (also shortly referred to as ACS) is the manifestation of a plaque rupture in a coronary artery.

The rupture or the erosion of an atherosclerotic plaque, with the subsequent formation of thrombus and occlusion of the artery may cause myocardial infarction and unstable angina (see, for a general reference, "New insights into atherosclerotic plaque rupture" D.M. Braganza and M.R. Bennett, Postgrad. Med. J. 2001; 77; 94-98).

An atherosclerotic event begins as a subendothelial accumulation of lipid laden, monocytes derived foam cells and associated T cells which form a non-stenotic fatty streak. With progression, the lesion takes the form of an acellular core of cholesterol esters, bounded by an endothelialised fibrous cap containing smooth muscle cells (VMSC) and inflammatory cells (both macrophages and T lymphocytes). Also presented in the advanced lesions are new blood vessels and deposits of calcium hydroxyapatite may also appear in advanced lesions (see as a general reference, "Coronary disease: Atherogenesis: current understanding of the causes of atheroma" Peter L. Weissberg, Heart 2000; 83; 247-252).

The extracellular lipid core of the plaque is composed of free cholesterol, cholesterol crystals and cholesterol esters derived from lipids infiltrated the arterial wall or derived from the dead foam cells. The lipid core may affect the plaque by causing stress to the shoulder regions of the plaque; in addition, the lipid core contains prothrombotic oxidized lipids and it is further impregnated with tissue factors derived from macrophages in which the lipid core materials are highly thrombogenic when exposed to circulating blood (see, for instance, "Mechanism of Plaque Vulnerability and Rupture" Prediman K. Shah, Journal of the American College of Cardiology 2003).

The stability of the plaque depends also upon the vascular smooth muscle cells (SMCs) content of the plaque, as they are capable of synthesising the structurally important collagens types I and III. In contrast, macrophages and others inflammatory cells may release matrix metalloproteinases (MMPs) which degrade collagen and extracellular matrix, thus potentially weakening the plaque (see, "New insights into atherosclerotic plaque rupture" D.M. Braganza and M.R. Bennett, Postgrad. Med. J. 2001; 77;94-98).

The structural components of the fibrous cap include matrix component such as collagen, elastin and proteoglycans, derived from SMCs. Said fibrous cap protects the deeper components of the plaque from contact with circulating blood and has been observed to thin out in the vicinity of the rupture (see, for example, "Mechanism of Plaque Vulnerability and Rupture" Prediman K. Shah, Journal of the American College of Cardiology 2003).

Ruptured plaques have been shown to have several histomorphologic features with respect to intact plaques. Therefore, when they are present, they are thought to indicate vulnerability to plaque rupture (see, for instance, "Mechanism of Plaque Vulnerability and Rupture" Prediman K. Shah, Journal of the American College of Cardiology 2003).

One of possible causes inherent to the plaque formation is thought to be caused by repeated injury to endothelium caused by the four "major" risk factors: smoking, hypertension, diabetes and hyperlipidaemia (high level of LDL and low level of HDL). Endothelial dysfunction following injury, moreover, plays a crucial role in plaque initiation, as lipids may pass more easily from the bloodstream into the *tunica intima.*

The rupture of a vulnerable plaque may occur either spontaneously, i.e. without occurrence of any of the above mentioned triggers or following a particular event, such as an extreme physical activity, a severe emotional trauma and stresses of different nature or acute infection.

Plaque rupture often leads to thrombosis with clinical manifestations of an ACS.

The thrombotic response to a plaque rupture is probably regulated by the thrombogenicity of the constituents exposed on the plaque; generally, the plaque rupture develops in a lesion with a necrotic core and an overlying thin fibrous cap heavily infiltrated by inflammatory cells. A luminal thrombus further develops due to the physical contact between platelets and the necrotic core (see, for example, "Pathologic assessment of the vulnerable human coronary plaque" F.D. Kolodgie et al. Heart 2004; 90; 1385-1391).

Rupture or erosion of the fibrous cap exposes the highly thrombogenic collagenous matrix and lipid core to circulation leading inevitably to platelet accumulation and activation. This in turn leads to fibrin deposition and thrombus formation which may result into vessel occlusion, the latter being not inevitable, such as in the case of silent episodes (see, for instance, "Coronary disease: Atherogenesis: current understanding of the causes of atheroma" Peter L. Weissberg, Heart 2000; 83; 247-252).

Until recently, atherosclerosis was thought of as a degenerative and slowly progressive disease causing symptoms through its mechanical effects on blood flow, while it is now understood to be a dynamic inflammatory process that is eminently modifiable. Recent researches on cellular and molecular events underlying development and progression of atherosclerosis, focus the attention on the dynamic interaction between the plaque components that dictates the outcome of the disease (see, as a general reference "Coronary disease: Atherogenesis: current understanding of the causes of atheroma" Peter L. Weissberg, Heart 2000; 83; 247-252).

There are contrasting data for a relation between coronary syndrome and several pathogens to be assessed.

In a prospective study (see, for example, "Impact of viral and bacterial infectious burden on long term prognosis in patients with coronary artery disease" Rupprecht H.J. et al., Circulation 2001, Jul 3; 104(1): 25-31) it was described the relation between stroke and 8 different pathogens (*Herpes simplex virus 1-2, Epstein-Barr, Cytomegalovirus, Haemophilus influenzae, Mycoplasma pneumoniae, Helicobacter pylori* and *Chlamydia pneumoniae*) in a group of 1018 patients; there was found an increase in mortality, related to the serum positivity for six to eight pathogens of 7% and 12.6 % respectively.

De Palma and his group ("Patients with Acute Coronary Syndrome Show Oligoclonal T-Cell Recruitment Within Unstable Plaque" De Palma et al. Circulation 2006,113: 640-646) conducted a study on the T cells *repertoire* recovered from blood sample and also directly from the coronary plaque of patients with acute coronary syndrome.

### Antibody structures

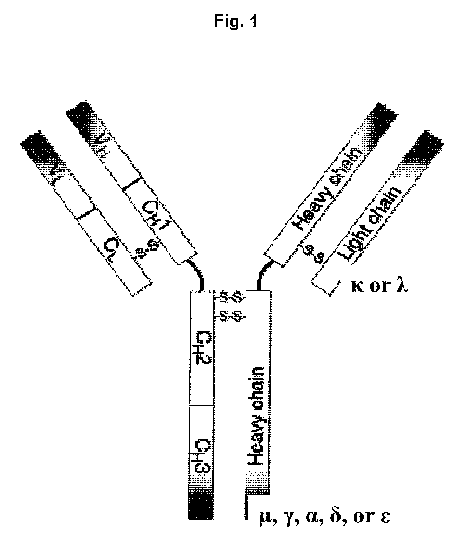

There exist five types of antibodies (also called immunoglobulins): IgG, IgA, IgD, IgM and IgE. The structure of IgG, depicted in Figure 1, comprises two light chains of a molecular weight of approximately 23 KDa and two heavy chains of about 53-70 KDa. The four chains being linked to each other by disulfide bonds in a "Y" configuration.

Heavy chains are classified as γ, η, α, δ and ε with some subclasses among them, while light chains are classified as either κ or λ.

Each heavy chain comprises a constant region and a variable region, the latter being located at the N-terminal end of the immunoglobulin molecule of approximately 100 amino acids in length.

In particular, the most variable part of the immunoglobulin (Ig) heavy and light chains is the third complementarity-determining region (CDR3), a short amino acid sequence which is formed by the junctions between the V-D-J gene segments. CDR3 is found in the variable domains of antigen receptor (e.g. immunoglobulin and T cell receptor) protein that complements an antigen.

The variability of the CDR3 portion is responsible of the elevated number of antibodies produced and which are specific for any antigens; said variability is determined by the rearrangement of the V, D and J minigenes that occurs in the bone marrow during the generation of mature B cells.

After this first rearrangement has occurred, when the mature B cell encounters an antigen, further hypermutational events are responsible for the increased affinity of the antibody for that specific antigen.

### Examples of objects encompassed by the present invention

The following are illustrative examples of the objects of the invention, that will be more apparent from the teaching of the whole disclosure.

It is a first object of the present invention the antibodies or fragments thereof corresponding to the amino acidic Sequence ID 2 and 4.

A second object of the present invention includes an expression vector, comprising one or more of the isolated polynucleotidic molecules of Sequence ID 1 and 3, as well as the complementary sequences thereof, encoding for the amino acidic sequences corresponding to ID 2 and 4 and the homologous sequences thereof.

A third object of the present invention includes an expression system comprising one or more of the isolated expression vector of the invention and a suitable host cell.

As further object of the present invention, there is provided a host cell comprising one or more of the expression vector of the present invention.

An additional object of the present invention includes a process for the production of recombinant antibodies or fragments thereof including the use of the expression system of the invention comprising one or more of the isolated polynucleotidic molecules of Sequence ID 1 and 3 as well as the complementary and homologous sequences thereof.

A further object of the invention encompasses the isolated recombinant antibodies or fragments thereof produced by the host cell comprising the expression vector of the present invention.

It is another object of the present invention an immunoassay including the use of one or more of the amino acidic sequences corresponding to Sequence ID 2 and 4 and the homologous sequences thereof.

In an additional embodiment of the invention, there is provided a therapeutic composition comprising the antibodies of the present invention or any fragments thereof and a therapeutic moiety linked thereto.

In a further embodiment of the invention, there is provided a diagnostic composition comprising the antibodies of the invention or fragments thereof linked to a diagnostic moiety.

It is a still further embodiment of the present invention a ligand that specifically binds the antibodies of the invention or to any fragments thereof.

A further object of the invention, a method for the screening of molecules for identifying those having the most binding affinity for the antibodies of the present invention or for any fragments thereof.

As an additional embodiment of the present invention there is an immunoassay, which includes the use of the ligand identified according to the present invention.

In a still additional embodiment of the invention, it is disclosed a therapeutic or diagnostic composition comprising the ligand of the present invention, covalently linked or otherwise functionally associated to a therapeutic or to a diagnostic moiety or entity.

An additional embodiment of the invention is represented by the use of immunosuppressant, immunomodulant or antinfective agents for the preparation of pharmaceutical compositions for the treatment of coronary diseases, such as the acute coronary syndrome or of immuno-related pathologies.

In a further embodiment of the invention, there is provided a method for the identification of the ethiologic agent responsible for the development of immuno-related pathologies.

### Brief description of the Figures

**Fig.1** is a schematic representation of the structure of an IgG antibody molecule and of a Fab fragment thereof.

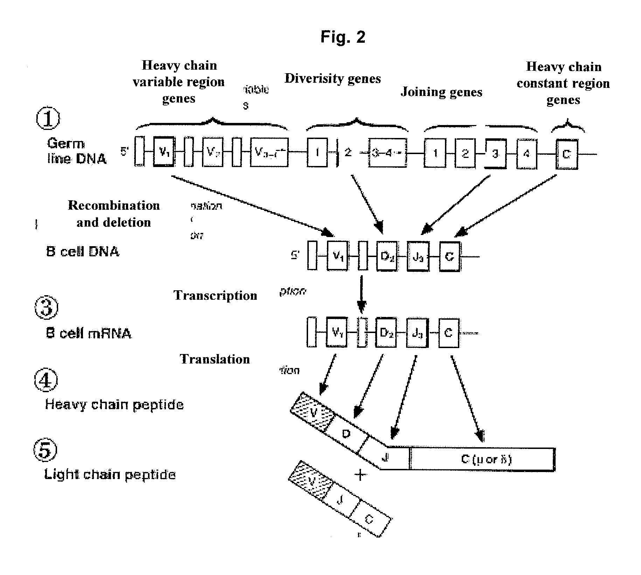

**Fig.2** represents the recombinant pattern for the production of antibodies.

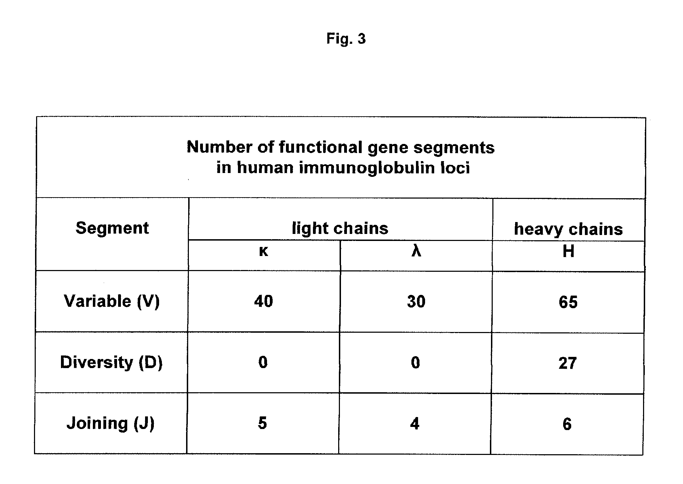

**Fig.3** represents the number of functional gene segments in human immunoglobulin *loci.*

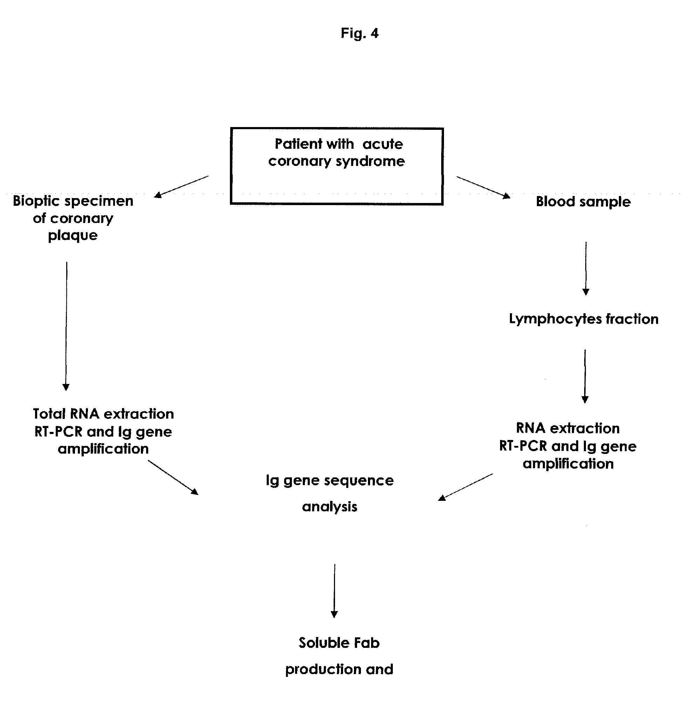

**Fig.4** is a schematic representation of the preparation of the antibodies or fragments thereof according to the present invention.
**Fig.5** shows the results of immunoaffinity selection by Biopanning on cell lysate (Hep-2), carotid lysate, artificial antigens.
**Fig.6** shows the ELISA results for Fab 68.

### Description of the invention

### Definitions

In the present invention, and unless otherwise provided, the term "isolated polynucleotide" or "isolated nucleotide" refers to a polynucleotide molecule, wherein polynucleotidic and nucleotidic, respectively, and polynucleotide and nucleotide are used alternatively with the same meaning, which is substantially free of any other cellular material or component that normally is present or interact with it in its naturally occurring environment, such as fragments of other nucleotidic or polynucleotidic sequences, proteins or other cellular component.

Unless otherwise provided, "complementary sequence" refers to the sequence which hybridizes to the sequence of interest under stringent conditions, resulting in two hydrogen bonds formed between adenine and tymine residues or three hydrogen bonds formed between cytosine and guanidine residues, respectively, and conservative analogs thereof having degenerative codon substitution or silent substitution, i.e. substitution of one or two or three consecutive nucleotides resulting in the same amino acid being coded due to the degeneracy of the genetic code.

The isolated polynucleotides within the meaning of the present invention, comprise, for instance, gene or gene fragments, exons, introns, mRNA, tRNA, rRNA, rybozyme, cDNA, plasmids, vectors, isolated DNA, probes and primers.

Unless otherwise indicated, the isolated polynucleotides of the invention, in addition to the specific ones described above, also comprise the complementary sequences thereto.

"cDNA" refers to the complementary DNA sequence, both single and double stranded and to any homologous sequence thereto and any fragment thereof, which codes continuously for an amino acidic sequence, i.e. its sequence is deprived of introns, and may be synthesized from isolated mRNA by retro-transcription techniques.

"Homologous sequence" within the meaning of the present invention refers to any sequence which is partially or almost identical to the sequence of interest; therefore, "homology" or "identity" of two or more sequences, comes from the factual measurement of the number of the same units, being those units nucleotides or amino acids, out of the total units componing said nucleotidic/amino acidic sequence, which occupy the same position. For example, 90% homology means that 90 of every 100 units making up a sequence are identical when the two sequences are aligned for maximum matching. Within the present invention, homologous sequences have an identity of at least 85%, preferably of 90%, more preferably of 95% and even more preferably of at least 99.5%.

"Conservative substitutions" of an amino is intended to be a substitution of an amino acid with another amino acid having the same properties, so that the substitution has no impact on the overall characterizing properties or functions of the peptide.

Examples of such conservative substitutions include the substitution of an amino acid with another amino acid belonging to the same group as follows:
(i) amino acids bearing a charged group, comprising Glutamine and Aspartic acid, Lysine, Arginine and Histidine;
(ii) amino acids bearing a positively-charged group, comprising Lysine, Arginine and Histidine;
(iii) amino acids bearing negatively-charged group, comprising Glutamine and Aspartic acid;
(iv) amino acids bearing an aromatic group, comprising Phenylalanine, Tyrosine and Tryptophan;
(v) amino acids bearing a nitrogen ring group, comprising Histidine and Tryptophan;
(vi) amino acids bearing a large aliphatic nonpolar group, comprising Valine, Leucine and Isoleucine;
(vii) amino acids bearing a slightly-polar group, comprising Metionine and Cysteine;
(viii) amino acids bearing a small-residue group, comprising Serine, Threonine, Aspartic acid, Asparagine, Glycine, Alanine, Glutamic acid, Glutamine and Proline;
(ix) amino acids bearing an aliphatic group comprising Valine, Leucine, Isoleucine, Metionine and Cysteine;
(x) amino acids bearing a small hydroxyl group comprising Serine and Threonine.

In the following disclosure, "CDR3" is a short sequence refers to the complementary-determining region, which is formed by the junctions between the V-D-J gene (in the heavy chain) or V-J gene (in the light chain) segments coding for an antibody. CDR3 is found in the variable domains that complements an antigen.

"Single clone" refers to a sequence coding for the CDR3 region of an antibody, which is able to specifically bind an antigen/epitope.

Sequences showing the same CDR3 are deemed to be produced by the same clone.

"Clonal variant" is intended to be any sequence, which differs by one or more nucleotide or amino acid, in presence of V region with identical mutations compared to the germline, identical VDJ or VJ gene usage, and identical D and J length.

"Replacement mutation" is intended to be a nucleotidic mutation which causes another amino acidic to be coded.

"Silent mutation" is intended to be a nucleotidic mutation which does not cause a change in the coded amino acid due to the degeneracy of the DNA.

An "expression vector" is intended to be any nucleotidic molecule used to transport genetic information.

An "isolated expression system" is intended to be a system for the expression of amino acidic molecules, and shall include one or more expression vectors comprising the nucleotidic sequences coding for one or more of the amino acidic molecules of the invention and a suitable host cell in which the one or more vectors are transfected.

"Host cell" as for the present invention is intended to be a cell comprising one or more expression vectors of the invention and which is capable of producing the corresponding coded amino acidic sequence or sequences or any fragments thereof, for example by expressing it on its surface.

"Antibodies" and "antibodies fragments" according to the present invention is intended to include whole antibodies, also referred to as immunoglobulin, of either type IgG, IgA, IgD, IgM or IgE, as well as any fragments thereof, such as proteolytic and/or recombinant fragments, like Fab fragments (produced upon digestion of Ig with papain), F(ab')₂ (produced upon digestion of immunoglobulin with pepsin), Fab', Fv, single chain antibodies (scFv) and single chain of antibodies, such as, for instance, heavy or light single chains.

"Ligand" within the present invention, is intended to be any agent that binds a recognized functional region of the antibody of the present invention or to any fragment thereof.

"Oligopeptide" according to the present invention is an amino acidic sequence comprising less than 50 amino acidic residues.

In the following description and unless otherwise provided, the "germline" sequence is intended to be the sequence coding for the antibody/immunoglobulin or of any fragment thereof deprived of mutations, therefore, the percentage of homology represents an indication of the mutational events which any type of heavy chain portion undergo after contact with an antigen; more in particular, said mutations involve the CDR3 portion of the antibody/immunoglobulin or of any fragment thereof.

The "R:S mutation" ratio refers to the ratio between replacing (R) and silent (S) mutations occurred in the FR or CDR3 portion of the antibody/immunoglobulin coding sequence.

Said ratio is higher for CDR3 than that of the FR sequence, as CDR3 undergoes an higher number of mutational event in order to adapt to the structure of the antigen. FR, in contrast, is a more conservative sequence, generally.

### p-value

"P-value" represents the significance of a mutational event.

In particular, the process of somatic hypermutation of rearranged V segments and the antigen selection of mutants with a higher affinity, allow the affinity maturation, in order to generate antibodies with improved binding properties to the antigen. This process leads to an accumulation of replacement mutations (R) in CDR regions, which are directly involved in the binding of antigen. On the contrary the silent mutations (S) accumulate in the FR regions, which are usually more conservative sequences in order to maintain the conformation of the antibody. In absence of the antigen selection, a random mutational process results in random distribution of R and S mutations in the sequence of both heavy and light chains of an antibody. However during the selection process, the R:S mutation ratio for CDR3 is usually higher than that of the FR sequence.

Therefore, the p-value, which is calculated by multinomial distribution model that the excess (as for CDR) or the scarcity (as for FR) of mutations occurred by chance, relates to the probability of an antigen selection process. A low p-value indicates that there is a high probability that the variability of the heavy and light chains compared to the corresponding germline sequence, is due to the antigen-driven affinity maturation of the antibody.

A significant p-value is intended to be below 5%.

According to its first embodiment, the present invention concerns polynucleotidic molecules comprising any one of the Sequence ID 1 and 3 and the complementary and homologous sequences thereto.

The polynucleotidic sequences of the present invention codes for the amino acidic sequences of antibodies or any fragments thereof which binds to an antigen or any fragment thereof possibly found in the coronary plaque.

Preferably, within the present invention, the isolated polynucleotides of the above first embodiment are cDNA molecules.

cDNA is obtained by retro-transcription from mRNA molecules according to the well-known procedures in the art.

According to the first object of the present invention, there are also provided amino acidic sequences corresponding to Sequence ID 2 and 4; as well as the homologous sequences thereof, and any sequences bearing conservative substitutions and fragments thereof.

As indicated, these definitions are intended to encompass analogous sequences, so as to include those sequences wherein, in the case of amino acid sequences, at least one or more amino acids are substituted by a derivative, such as the corresponding D-isomer or, for example, the corresponding sulphated, glycosylated or methylated amino acid; or one or more and up to 10% of the total amino acids making up a sequence may be substituted by a derivative thereof, such as, for example, cysteine may be substituted by homocysteine. There are also included sequences bearing conservative substitutions.

According to the present invention, there are also included the polynucleotidic sequences coding for antibodies or for any fragments thereof according to the first embodiment of the invention and having homology of at least 80%, preferably of at least 90%, more preferably of at least 95% and even more preferably of at least of 97% compared to the germline, when using a database available in ImMunoGeneTics (available through the web site http://imgt.cines.fr).

In addition, as for the first object of the present invention, hypermutated amino acidic sequences are also encompassed.

Accordingly, there are also included the polynucleotidic sequences coding for the amino acidic sequences having a p-value of the CDR3 portion less than 5%, preferably less than 2%, more preferably less than 1% and even more preferably less than 1‰ and the coded amino acidic sequences thereof.

As set hereinbefore, according to the present invention, there is included the synthesis of cDNA molecules, which is performed from mRNA isolated from a suitable sample of the active coronary plaque of a patient.

For the purpose of the present invention, said suitable samples of the active coronary plaque includes a sample of the coronary plaque taken immediately after an infarction event, i.e. so-called "fresh-sample" or, alternatively, a sample may be taken and conserved under liquid nitrogen for a suitable period of time so as not to impair nor alter its histological properties and be further analysed.

For the purpose of the present invention, patients with acute coronary syndrome (ACS) have been selected, which have experienced a typical chest pain occurring less than 48 hours from hospital admission or ECG changes suggesting myocardial damage. In order to exclude possible confusing factors, patients with recent infectious diseases, immunologic disorders, immunosuppressive therapy or neoplastic diseases have been excluded.

Isolation of mRNA molecules from the above suitable samples, i.e. both from coronary plaque and peripheral blood, is carried out according to well-known methods. For a general reference, see, for instance Molecular cloning. Sambrook and Russell. Cold Spring Harbor Laboratory Press Cold Spring Harbor, New York. Third Edition 2001*.*

According to the second embodiment, the expression vector of the invention is selected from the group comprising for example, plasmid, cosmid, YAC, viral particle, or phage and comprises one or more of the polynucleotidic sequences according to the first embodiment of the invention; in a preferred aspect, the expression vector is a plasmid, comprising one or more of the polynucleotidic sequences according to the first embodiment of the invention.

In a most preferred embodiment of the invention, the expression vector, i.e. a plasmid, comprises one of more of the polynucleotidic sequences ID 1 and 3.

Expression vectors ordinarily also include an origin of replication, an operably linked, i.e. connected thereto in such a way as to permit the expression of the nucleic acid sequence when introduced into a cell, promoter located upstream the coding sequences, together with a ribosome binding side, an RNA splice site, a polyadenylation site and a transcriptional sequence. The skilled artisan will be able to construct a proper expression vector and, therefore, any proper expression vector according to the selected host cell; for example, by selecting a promoter which is recognized by the host organism.

In an even more preferred embodiment, the expression vector of the present invention is represented by the vector described by Burioni et al. (Human Antibodies 2001; 10 (3-4): 149-54).

The isolated expression system according to the third embodiment of the invention may comprise a single expression vector, which comprises one or more of any one of the polynucleotidic sequences of the invention.

Alternatively, the above expression system may comprise two or more expression vectors, each of them comprising one or more of any one of the polynucleotidic molecules of the invention.

For example, an expression vector may comprise a polynucleotidic molecule of the invention coding for the light chain of an antibody or fragment thereof and a second expression vector may include a polynucleotidic molecule of the invention coding for the heavy chain of an antibody or fragment thereof.

In an embodiment of the invention, the expression system comprises a single expression vector including one or more of the polynucleotidic molecules comprising Sequence ID 1 to 3 and coding for the amino acidic sequences and corresponded to the Sequence ID from 2 to 4 and any homologous sequence thereto.

In a preferred embodiment of the invention, the expression system comprises one expression vectors comprising the polynucleotidic sequence coding for a light chain, i.e. the Sequence ID from 3 and a second polynucleotidic sequence coding for a heavy chain, i.e. being selected the Sequence ID 1.

The preparation of the expression vector comprised into the expression system of the invention, includes the insertion of the appropriate nucleic acid molecule o molecules into one or more vector or vectors, which generally comprises one or more signal sequences, origins of replication, one or more marker genes o sequence, enhancer elements, promoters, and transcription termination sequences according to methods well-known in the art.

For a general reference to said procedure, see, for instance Phage display, Cold Spring Harbor Laboratory Press. Cold Spring Harbor, New York*.*

For instance, the sequences coding for the heavy chain of the present invention are inserted into the expression vector with a Flag o a six-Histidine tail, for being easily detectable.

The host cell according to a forth embodiment of the present may be, for instance, a prokaryotic cell or a eukaryotic cells.

Suitable prokaryotic cells include gram negative and gram positive and may include, for example, *Enterobacteriaceae* such as *Escherichia,* e.g. *E. coli, Enterobacter, Erwinia, Klebsiella, Proteus, Salmonella,* e.g. *Salmonella typhimurium, Serratia,* e.g. *Serratia marcescans,* and *Shigella,* as well as *Bacilli* such as *B. subtilis* and *B. licheniformis, Pseudomonas* such as *P. aeruginosa,* and *Streptomyces.* For example, publicly available strains which may be used are, for instance, *E. coli K12* strain *MM294 (ATCC 31,446); E. coli X1776 (ATCC 31,537); E. coli* strain *W3110 (ATCC 27,325)* and *K5 772 (ATCC 53,635)* or *E. coli* XL1-Blue, which represents the preferred *E. coli* strain.

In addition to prokaryotes, eukaryotic microbes such as filamentous fungi or yeast are suitable host cells. *Saccharomyces cerevisiae,* also known as common baker's yeast, is commonly used; other yeast are, for instance, *Saccharomyces, Pichia pastoris,* or *Kluyveromyces* such as, for example, *K. lactis, K. fragilis, K. bulgaricus, K. wickeramii, K. waltii, K. drosophilarum, K. thermotolerans,* and *K. marxianus, Schizosaccharomyces,* such as *Schizosaccharomyces pombe, yarrowia, Hansenula, Trichoderma reesia, Neurospora crassa, Schwanniomyces* such as *Schwanniomyces occidentalis, Neurospora, Penicillium, Tolypociadium, Aspergillus* such as *A. nidulans, Candida, Torulopsis* and *Rhodotorula.*

In addition, suitable eukaryotic cells used for the preparation of the expression system may be derived from multicellular organisms as well, such as from invertebrate cells or plant cells. Plant cells include, for instance, *Agrobacterium tumefaciens* and *Nicotiana tabacum.* In addition, insect cells may be used, which include, for instance, *Drosophila S2* and *Spodoptera Sf9.*

Conversely, mammalian host cell include Chinese hamster ovary (CHO) and COS cells. More specific examples further include monkey kidney CVI line transformed by SV40 (COS-7, ATCC CRL 1651); human embryonic kidney line, Chinese hamster ovary cells/-DHFR, mouse sertoli cells, human lung cells (W138, ATCC CCL 75); human liver cells (Hep G2, HB 8065); and mouse mammary tumor (MMT 060562, ATCC CCL51).

The selection of the appropriate host cell is deemed to be within the knowledge of the skilled person in the art, i.e. prokaryotic cells may be used for the preparation of antibodies fragments such as Fabs, while for the preparation of whole antibodies such as IgG, eukaryotic cells like yeasts may be employed.

Methods for cell transfection and transformation in order to prepare the above disclosed host cell comprising the above expression system depends upon the host cell used and are known to the ordinarily skilled artisan.

For example, treatments with calcium or electroporation are generally used for prokaryotes, while infection with *Agrobacterium tumefaciens* is used for transformation of certain plant cells. For mammalian cells, calcium phosphate precipitation may be used as disclosed by Graham and van der Eb, Virology, 52:456-457 (1978).

However, other methods for introducing polynucleotidic sequences into cells, such as, for example, nuclear microinjection, electroporation, bacterial fusion with intact cells, or polycations, may also be used.

Host cells, in addition, may also be transplanted into an animal so as to produce transgenic non-human animal useful for the preparation of humanized antibodies or fragments thereof. A preferred non-human animal includes, for instance, mouse, rat, rabbit, hamster.

The production of recombinant antibodies and fragments thereof as for an embodiment of the invention is performed according to known methods in the art and includes the use of the isolated polynucleotidic sequences of the invention.

In particular, said method includes the steps of:
a) isolating mRNA from a suitable sample of the coronary plaque;
b) performing reverse transcription in order to obtain the corresponding cDNA;
c) preparing an expression system comprising the one or more cDNA molecule or molecules obtained from step b) and any one of the above disclosed suitable host cells;
d) culturing the host cell under suitable growth conditions;
e) recovering the produced antibodies or any fragments thereof; and
f) purifying said antibodies or any fragments thereof.

In particular, steps a) to f) are performed according to known methods in the art as it will be apparent from the following Examples.

In order to assess the influence on the results obtained by statistically occurring mutations or other mechanism different from those involved in the maturation of B-cells of the coronary plaque, cloning and sequencing is also performed on a small portion of a gene having a conserved region. Accordingly, as internal reference, β-globin gene is chosen; in particular, standard β-giobin L48931 is used.

Therefore, it is a further object of the present invention, the isolated recombinant antibodies and fragments thereof produced by the host cell of the present invention and according to the method disclosed above, include immunoglobulin (shortly referred to as Ig) of the IgG type, while "fragments thereof" preferably include Fab fragments of IgG.

According to the present invention, there are also included the amino acidic sequences coding for antibodies or for any fragments thereof which may be produced according to the process above disclosed and having homology of at least 80%, preferably of at least 90%, more preferably of at least 95% and even more preferably of at least of 97% compared to the germline, when using a database available in ImMunoGeneTics (available through the web site http://imgt.cines.fr).

In addition, there are also included the amino acidic sequences having a p-value of the CDR3 portion less than 5%, preferably less than 2%, more preferably less than 1% and even more preferably less than 1‰.

According to another object of the invention, there is provided an immunoassay, which comprises the use of the antibodies or of any fragments thereof produced according to the present invention.

Immunoassays are test based on the formation of an antigen/antibody complex and can be either competitive or non-competitive.

Competitive immunoassays include the testing of unknown samples containing a particular antigen which competes for the binding to the antibodies with another but labelled antibody; therefore, the response is inversely proportional to the concentration of the antigen in the unknown sample.

Conversely, non-competitive immunoassays, also called "sandwich assays", include the use of an immobilized antibody, bound by an antigen, thus forming a complex which is targeted by a labelled antibody; the result of said methods is, therefore, directly proportional to the concentration of the antigen.

Widespread used immunoassays include, for example, RIA (Radio Immune Assay), Western Blot, ELISA (Enzyme-linked Immunosorbent Assay), immunostaining, immunoprecipitation, immunoelectrophoresis, immunofluorescence, luminescent immunoassay (LIA), immunohystochemistry, which are routinely used in lab practise.

A preferred immunoassay according to the present invention is an ELISA test.

ELISA is a well-established biochemical technique, which allows the detection and further quantification of biomolecules, such as antibodies or fragments thereof, antigens, proteins, hormones and other organic molecules, in a given sample; preferably, according to the present invention, the above mentioned ELISA test is used for the detection of a specific antigen.

ELISA test, in particular, may include the use of two antibodies, one of which, the first antibody, is selective for the molecule of interest, i.e. the antigen, and it is immobilized onto an ELISA plate. A mixture possibly containing said molecule of interest is added, incubation for a suitable and sufficient time is allowed, then a first washing is performed in order to remove unbound material. The secondary antibody coupled to an enzyme and specific for the complex formed between the molecule of interest and the first antibody is further added. There follows a second step of washing of the ELISA plate and the addition of a chromogenic substrate. The resulting variation in colour may be assessed by spectrophotometric techniques and is directly related through a colorimetric standard curve to the quantity of the complex formed and thus to the concentration of the molecule of interest present in the sample.

Samples to be tested by the above immunoassay of the invention are, for example, samples of the unstable coronary plaque taken from patient immediately after an infarction event, i.e. a so-called "fresh" sample as said before, or a sample which has been conserved under liquid nitrogen after being taken; alternatively, it may consist of a sample of whole blood or serum.

The immunoassay test according to the present invention represents a valuable diagnostic tool, when included in programs for the screening of either the population at risk or not of developing acute coronary syndrome (ACS) or other coronary diseases.

As for an additional embodiment of the invention, there is disclosed a therapeutic composition comprising the antibodies or any fragments thereof of the present invention and a therapeutic moiety covalently linked thereto.

Said therapeutic composition is able to selectively target a therapeutic agent to the coronary plaque site.

Well-known advantages of said targeted composition include, among others, the possibility of reducing the quantity of active principle to be administered, thus reducing the potentially side effects thereof.

For said purpose, therapeutic moieties may include as non limiting examples, radionuclides, drugs, hormones, hormone antagonists, receptor antagonists, enzymes or proenzymes activated by another agent, autocrines or cytokines, antimicrobial agents; toxins can also be used.

Drugs and prodrugs are included as well.

A further embodiment of the invention relates to a diagnostic composition comprising the antibodies of the invention or any fragment thereof linked to a diagnostic moiety for the visualisation of the coronary plaque site.

The diagnostic compositions according to the present invention comprise the antibody or any fragments thereof, produced according to the present invention, covalently linked to at least one diagnostic moiety in order to selectively target the coronary plaque site and thus allowing its localization.

Therefore, it will be possible to precisely localise the site where the coronary plaque developed and to even better understand the extent of the occurred lesion to the vase. In addition, this represents a very useful tool before removal of the plaque by surgery.

Diagnostic moieties allow the detection by the visualising techniques used in the field of medicine, such as, for example, MRI (magnetic resonance imaging), CT (computer tomography), ultrasound, ecography, x-rays, and other diagnostic techniques within the knowledge of the skilled person in the art.

The kind of diagnostic moiety will be selected according to the diagnostic technique to be used.

According to a still further object of the present invention, there are provided ligands, that is to say, molecules which do bind selectively to the antibodies or to any fragments thereof.

The ligand or ligands of the present invention may also be an agent that binds any surface or internal sequences or conformational domains or any other part of the target antibody or fragments thereof. Therefore, the "ligands" of the present invention encompass agents that may have no apparent biological function, beyond their ability to bind the target of interest.

Accordingly, proteins, peptides, polysaccharides, glycoproteins, hormones, receptors, cell surfaces antigens, antibodies or fragments thereof such as Fab fragments, F(ab')2, Fab', Fv and single chain antibodies (scFv) or even anti-idiotype antibodies, toxins, viruses, substrates, metabolites, transition state analogs, cofactors, inhibitors, drugs, dyes, nutrients, growth factors, etc., without limitation, are included as well within the above definition.

In a preferred embodiment, the ligand of the present invention is an oligopeptide as above defined; preferably is a peptide comprising 4 to 12 amino acids, more preferably is a peptide comprising 4 to 10 and even more preferably is a peptide comprising 6 to 8 amino acids.

The identification of the ligands may be performed by screening tests on libraries of compounds. In particular, according to the present invention, said identification includes the use of the antibodies provided by the present invention or of any fragments thereof.

A method for the identification of ligands to the antibodies of the present disclosure or to any fragments thereof, therefore, represents a further object of the invention.

For instance, said method may include the binding of the antibodies or fragments thereof onto a solid phase, for example through a streptavidin-biotin linkage, followed by contacting the molecules to be tested with the thus prepared solid phase, so as to allow them binding to the complementary antibodies and then washing to remove unbounded material; finally, the extend of the binding can be determined by various methods such as, for instance, an ELISA test.

Preferably, said ELISA test is one wherein a first antibody or a fragment thereof, being selected from those of the present invention, is linked to a solid phase, for instance, by a biotin/streptavidin linkage, then a mixture containing the molecules to be tested is added, incubation is allowed for a suitable period of time, followed by removal of unbound material by washing. After that, the secondary antibody is admixed and incubation is allowed again. The molecules showing the highest affinity for the antibodies of the invention or for any fragments thereof may thus be isolated, identified and quantified according to well-known methods such as, for instance, by colorimetric measurements.

Alternatively, as for an additional embodiment of the present invention there is provided an immunoassay including the use of a ligand identified according to the present invention.

Said immunoassay may be any one of the immunoassays already mentioned above as for the second object of the invention.

For example, an immunoenzymatic test as for the claimed invention may be an immunohystologic assay as further detailed in Example 11.

The above immunohystologic assay can be performed in order to investigate the presence inside the plaque of the ligands identified and disclosed in the present invention according to the above embodiments.

In a still additional embodiment of the invention, there is disclosed a therapeutic composition comprising a ligand identified by the above method of the invention and covalently linked to a therapeutic moiety.

A therapeutic moiety for said purpose may be any one of those already described above.

In particular, the therapeutic composition thus provided may selectively target a therapeutic agent to the coronary plaque site.

There is also disclosed a diagnostic composition comprising a ligand identified by the above method of the invention and covalently linked to a diagnostic moiety.

A diagnostic moiety for said purpose may be any one of those already described above.

As for an additional embodiment of the invention, there is claimed the use of immunosuppressant compounds for the preparation of a pharmaceutical composition for the treatment of coronary diseases, such as the acute coronary syndrome (ACS) or of immuno-related pathologies.

Immuno-related pathologies include pathologies wherein the physiologic mechanisms triggering and controlling the immuno-responses are altered.

Immunosuppressant compounds may be selected from the group comprising by way of non limiting example, glucocorticoids, alkylating agents, antimetabolites, methotrexate, azathioprine and mercaptopurine, cytotoxic antibiotics such as dactinomycin, anthracyclines, mitomycin C, bleomycin, mithramycin, ciclosporine, interferons, opioids, TNF binding protein, mycophenolate, small biological agent; in addition, monoclonal and polyclonal antibodies are comprised.

In a further embodiement, the present invention provides for a method for the identification, demonstration and characterization of a local antigen-specific and antigen-driven stimulation of the immune system, providing useful details that can be used for the identification of the aetiopatology, for the definition of targets and for the design of immunotherapy and immunoprophylaxis.

In particular, said method includes the steps of testing the affinity of the antibodies of the present invention or of any fragments thereof for pathogenic agents potentially responsible for the development of the coronary disease.

With the aim of better understanding of the present invention, and without posing any limitation to it, the following Examples are given.

### Example 1: Sample collection

A sufficient amount of tissue is obtained from an atherosclerotic plaque of a patient with acute coronary syndrome undergoing coronary atherectomy and it is stored in liquid nitrogen.

### Example 2: mRNA extraction

The plaque taken according to Example 1 is homogenized and the total mRNA is extracted according to conventional methodologies using a commercial kit for the extraction of mRNA (Rneasy kit, Qiagen, Germany) and according to the instructions provided by the manufacturer.

### Example 3: mRNA retrotranscription

Reverse transcription of mRNA from the coronary plaque sample obtained as from Example 2 is performed using a commercial kit for the retrotranscription of mRNA, Superscript III RT (Sigma-Aldrich, St Louis, Missouri) according to the manufacturer's instruction. The cDNA synthesis is performed according to standard procedures from the total mRNA primed with oligo(dT).

### Example 4: Amplification of cDNA sequences

1 µl of cDNA obtained from the Example 3 undergo polymerase chain reaction. The reverse primers are designed in order to anneal to the segments of sequences coding for the constant region of heavy and light chains respectively. The forward primers are "family specific" and are designed to correspond to the 5' end of the heavy and light chain genes in the first framework region; see, as a reference, Phage display, Cold Spring Harbor Laboratory Press. Cold Spring Harbor, New York. Third Edition 2001*.* For the heavy chains, primers specific for IgG1 and IgG2 isotypes are used, whereas for the light chains primers specific for K isotype are used. Amplification round is conducted with the following thermal profile: 94°C for 15 seconds, 52°C for 1 minute and 72°C for 90 seconds. The reaction is conducted for 35 cycles. A negative control (the same mixture without DNA) and a positive control (a known sequence is inserted) are included in each reaction. The PCR product is analyzed by electrophoresis in a 2% agarose gel containing ethidium bromide. The reaction yields a ≅ 650 bp band corresponding to the light chains, and a 700 bp corresponding to the heavy chains. The amplicon, i.e. the product of the PRC process) is extracted from the gel with the use of a commercial kit for the extraction of DNA (QIAquick gel extraction kit; Qiagen, Germany) according to the manufacturer's instructions. Finally, the PCR products are recovered as per standard methods.

### Example 5: Molecular cloning and combinatorial antibody Fab fragment phage-display minilibrary

The PCR products of heavy and light chains that make the Fab (variable region and the CH1 domain) amplified from a human coronary plaque according to the previous Examples are cloned into a phagemidic vector (pRB32) that allows the combinatorial generation o heavy and light chain pairs and exposes (phage display) onto the external phage surface the Fab fragment codified by the DNA contained into the viral particle. This is obtained by fusing the heavy chain fragment with a phage M13 membrane protein.

This allowed the generation of a combinatorial antibody Fab fragment phage-display minilibrary.

### Example 6: Cell lysate preparation

Infected and unifected MRC5 and Hep-2 cell lysates are obtained after 5 days after infection with HCMV (MOI 0,1). Hep-2 (ATCC CCL-23) cells are grown in E-Mem (Invitrogen 0820234DJ) supplemented with Antibiotic/Antimycotic Solution (Invitrogen, Antibiotic/Antimycotic Solution, liquid 15240-062) and 10% FBS. Cells are regularly split 1:10 every 5 days. Five million cells are washed in PBS and lysed by using RIPA buffer (50mM Tris HCL ph8+ 150mM NaCl + 1% NP-40+ 0.5% NA deossicolate+ 0.1 %SDS) .

### Example 7: Immunoaffinity selection by Biopanning on cell lysate (Hep-2), carotid lysate, artificial antigens

The night before biopanning, coat an ELISA plate O/N at 4°C with 50 µL/well of antigen(s) (100 ng/well) solution in coating buffer (0.1 M Bicarbonate pH=8.6). After washing with deionised H₂O wells are blocked completely with PBS/BSA 3%, sealed and incubated in a humidified container for 1 hour at 37°C. Fifty µL phage suspension are added to each well (total of about 1011 PFU) and incubated for at least 2 hours at 37°C after sealing the plate. Phage is removed from every well (and kept for titration), washed 10 times with PBS/Tween 0.05%. After intensive washing, phages bound to the antigen aere eluted by washing each well with 50 µl of Elution Buffer (0.1 M HCI, adjusted with glycine to pH=2.2, BSA 1mg/ml) and adding to the eluent 3 µl of 2M Tris base. After the elution, 2 ml of fresh XL-1 blue are infected with the eluted phage. After an incubation at 37°C for 15 min, 10 ml of 37°C-prewarmed Superbroth (SB) (20 µg/ml ampicillin and 10 µg/ml tetracycline) are added. After incubation for 1 hour at 37°C on a shaker, 100 ml of SB containing 100 µg/ml Amp and 10 µg/ml Tet are added to each 10 ml-culture and incubated for 1 hour at 37°C on a shaker. An helper phage VCS M13 (total of 1012 PFU) is used to infect the culture and incubated on the shaker for 2 hours at 37°C. After addition of kanamycin (µg/ml) culture are incubated on a shaker O/N at 30 °C.

The day after cells are spin down at 6000 RPM (Sorvall SS34), 4°C for 15 minutes and PEG8000 (Sigma-Aldrich) is added to the supernatant to a final volume of 20 ml. Phages are let precipitate on ice for 30 minutes and then centrifuged at 11,000 RPM (Sorvall SS34) for 20 min. at 4 °C). Phages are then resuspend in 2 ml PBS/BSA 1 %, and stored at 4 °C for subsequent biopanning rounds.

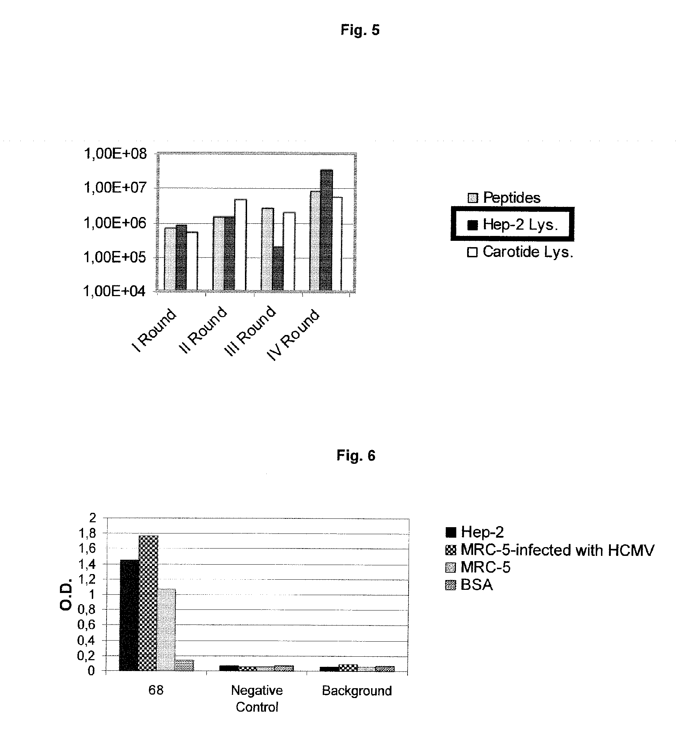

After each elution and at every round of selection, eluted phages are titered by plating 1µl and 0.1µl on LB plates (10-4 and 10-5 dilution of total) of the phage infected bacterial population. By titrating eluted phages after each round of selection allows to evaluate the efficacy of the enrichment procedure. The procedure is conducted for four times allowing enrichment of selected population. Results are shown in Fig. 5.

### Example 8: Analisys of selected clones after biopanning procedure on Hep-2 and artificial antigens

In all bio-panning procedures, the Fab of the invention is selected and it is predominant in the recovered phage population after the fourth round.

### Example 9: Sequencing

The sequences obtained according to the previous Examples are sequenced in for quantitative and qualitative analysis.

### 9.1) Heavy and light chain sample processing

A sample of clones of heavy and light chains obtained from coronary plaque sample obtained according to the previous Examples 4, respectively, is picked up in order to be sequenced by Big Dye chemistry and analyzed using ABI PRISM 3100 sequencer.

The obtained sequences are individually aligned to the germline segments using a database available in ImMunoGeneTics (available through the web site http://imgt.cines.fr), in order to identify the V,D,J and V and J genes recurrence as for the heavy and light chains respectively, the homology level with the germline and the extent of somatic mutations. CDR3 sequence identity is used for identifying the clones; as mentioned above, sequences with identical CDR3 are deemed to come from the same clone.

The polynucleotidic sequence from coronary plaque sample obtained according to the above Example 4 for the heavy chain correspond to Sequence ID 1 and coding for the amino acidic sequence corresponding to Sequence ID 2. The polynucleotidic sequence from coronary plaque sample obtained according to the above Example 4 for the light chain correspond to Sequence ID 3 and coding for the amino acidic sequence corresponding to Sequence ID 4.

### 9.2) B-globin sequence: internal reference

The analysis of five clones shows that the obtained sequence of β-gtobin is identical to the sequence present in database, thus demonstrating that no mutational event was due to the process variabilities.

### 9.3) Light chain from coronary plaque sample

The results of the sequencing of clone obtained from the coronary plaque sample according to Example 4 are shown in the following Table I for each clone V, D and J gene column report the type of sequence found to code for the V, D and J variable portion of the heavy chain, respectively. Homology percentage refers to the percentage of homology between the sequence cloned from the coronary plaque sample and the sequence of the corresponding germline sequence as above disclosed.

**Table I**

| # Clone | VK gene | JK gene | Homology (%) | R:S mutations | | p-value | |
|---|---|---|---|---|---|---|---|
| | | | | FR | CDR | FR | CDR |
| 8 | V3-15*01 | J4*01 | 94,90 | 3/2 | 5/2 | 0,00197 | 0,00091 |

### 9.4) Heavy chain from coronary plaque sample

The same procedure adopted for the analysis of the sequences of the light chains is repeated for the sequence of the heavy chains obtained according to Example 4.

The results are shown in the following Table II.

**Table II**

| # Clone | VK gene | DK gene | JK gene | Homology (%) | R:S mutations | | p-value | |
|---|---|---|---|---|---|---|---|---|
| | | | | | FR | CDR | FR | CDR |
| 6 | V5-51*03 | D6-13*01 | J5*01 | 82,95% | 20/16 | 8/1 | 0,0047 | 0,1848 |

Therefore, as clone 6 of the sequence amplified from the plaque show an higher divergence from the germline sequence, they are selected in order to be expressed together with the light chain 8.

### 9.5) Results

The above data show that both heavy and light chain from coronary plaque sample have an oligoclonal pattern and a characteristic VDJ and VJ gene pattern, respectively.

### Example 10: Preparation of the expression system with sequences from coronary plaque sample and transformation of host cells

Clone 8 of the light chain (corresponding to Sequence ID 3) and clone 6 of the heavy chain (corresponding to Sequence ID 1) of the coronary plaque sample are selected to be transfected into the expression vector for the preparation of the soluble Fab fragments according to the following procedure.

Gene encoding for the light chain selected according to the above Example 6 and corresponding to Sequence ID 3 is transferred into the expression vector pRB/expr and following the procedure disclosed by Burioni et al. Hum. Antibodies. 2001;10(3-4):149-54.

In the expression vector comprising the gene coding for the selected light chain is further introduced the gene coding for the heavy chain corresponding to the clone 6 (corresponding to Sequence ID 1) following the same procedure disclosed by Burioni et al. Hum Antibodies. 2001;10(3-4):149-54.

The expression vector is introduced into the *E.coli XL-1 Blue* for the expression of soluble Fabs.

In particular, 10 ml of SB (Super Broth, Becton, Dickinson, New Jersey) with ampicillin (100 ng/ml, Sigma-Aldrich, St Louis, Missouri) and tetrayicline (10 ng/ml, Sigma-Aldrich, St Louis, Missouri) is inoculated with a single bacterial colony from a fresh plate and incubated overnight at 37°C in an orbital shaker After that, 2.5 ml of this colture is inoculated into 1 liter of SB/amp-tet (the above mixture of SB, ampicillin and tetracyclin) into a 5 liter flask and allowed to grow until an Optical Density (OD₆₀₀) of approximately 1.0. Then IPTG (isopropyl-beta-D-thiogalactopyranoside; Biorad, California) is added up to a final concentration of 1 mM and the bacterial culture are incubated overnight at 30°C in the orbital shaker. Thus, bacteria are centrifuged at 3000 rpm for 20 minutes at 4°C and the pellets are resuspended in 10 ml PBS. Subsequently, 50 µl PMSF (from a stock solution of 100 mM) is added in order to inhibit the proteases and bacteria are sonicated three times in ice, 3 minutes for each run. The bacterial culture is centrifuged at 18000 rpm for 45 minutes at 4°C and the supernatant is filtered carefully with a 0.22 µm diameter membrane (Millipore®). Meanwhile, the column is washed with 10 volumes of PBS and subsequently the filtered supernatant is added slowly to the column. After washing with at least 30 volumes of PBS, Fabs are eluted with 100 mM glycine/HCI pH 2.5. 10 fractions are collected (each one of about 1ml) and immediately neutralized with Tris 1M pH 9.

Purified Fabs are tested in SDS-PAGE gel in non-reducing conditions showing a single band of approximately 50 kDa.

Fabs are quantified comparing the relative band with at least two different standard concentrations of BSA.

### Example 11: Immunohystologic assay

A fresh sample of plaque is frozen in liquid nitrogen and sectioned using a cryostat. Sections 5 µm thick are fixed with ice-cold acetone and blocked with a serum blocking solution (2% serum, 1%BSA, 0.1% Triton X-100, 0.05% Tween 20) for 1 hour at room temperature. The fixed sections are probed with the Fabs produced and identified according to the present invention, at an appropriate dilution, and incubated for 2 hours at room temperature. Sections are washed five times with PBS and an appropriate dilution of a FITC (fluorescein isothiocyanate)-conjugated secondary anti-human Fab (Sigma-Aldrich, St Louis, Missouri) is added. After 30 minutes at room temperature, sections are washed again and the complex ligand/antibody thus formed is detected with a fluorescence microscope.

### Example 12: Antibody screening of phage library

Panning of the random phage-displayed peptide library expressing dodecapeptides at the N-terminus of cplll coat protein of the filamentous phage M13 (Ph.D.-12^{™} Phage Display Peptide Library Kit, Catalog #E8110S, New England Biolabs, Beverly, Massachusetts) is performed according to the manufacturer's instructions using Fab-coated high-binding 96-well ELISA plates (Costar 96w polystyrene 1/2 area flat bottom HI-binding flat bottom, cat #3690).

In order to remove phages binding to antibody conserved regions, a negative selection is performed from the second round of panning by combining the amplified phages with 25 µg of a pool of human standard IgG (Endobulin, A.T.C J06BA02, Baxter S.p.A.) for 1 hour at 37°C.

Four rounds of selection are performed as described above, panning the amplified phage on Fabs produced and identified according to the present invention and the same pool of standard IgG used for the negative selection.

### Example 13: Peptide screening and DNA sequence analysts

All the phages obtained as from Example 11 are used to infect *E.coli* strain ER2537 and randomly picked single plaques are screened in enzyme-linked immunoassay on Fabs produced and identified according to the present invention and the pool of standard IgG.

Antigen-coated plates (Costar 96w polystyrene 1/2 area flat bottom HI-binding flat bottom, cat #3690) are washed and blocked with a solution of PBS/BSA 1% for 1 hour at 37°C; 50 µl of 10⁸ phages per milliliter are added and incubated for 2 hours at 37°C.

Plates are washed 10 times with PBS (0.1% Tween-20; Sigma-Aldrich, St Louis, Missouri); afterward, 50 µl of a 1:3000 dilution in PBS of a HRP-conjugated anti-M13 antibody (GE Healthcare 27-9411-01) is added.

After 2 hours at 37°C plates are washed P with PBS (0.5% Tween-20; Sigma-Aldrich, St Louis, Missouri), specific bound phages are detected by adding 100 µl of substrate (Sigma-Aldrich, St Louis, Missouri) and plates are read for an Optical Density of 450nm after 30 minutes at room temperature.

Positive clones showing an OD₄₅₀ₙₘ value > 1 on Fabs of the present invention and OD₄₅₀ₙₘ value < 0.3 on pool of IgG are scored as positives and evaluated by sequence analysis using the software Pepitope http://pepitope.tau.ac.il/index.html. From peptide sequence analisys conserved aminoacidic positions are identified and four peptides are selected on the basis of the amount of consensus residues present in their sequences.

Four peptides have been identified, and the related sequences corresponding to Sequence ID from 5 to 8.

### Example 14: Enzyme-Linked ImmunoSorbent Assay

Elisa plates (Costar 96w polystyrene 1/2 area flat bottom HI-binding flat bottom, cat #3690) are coated with serial dilution of Hep-2 Lysate, MRC5 and MRC5 infected with HCMV (1000 ng, 200 ng, 40 ng and 8 ng in PBS) overnight at 4°C. After blocking with PBS+BSA3% for 2 hours at 37°C, serial dilutions of Fab 68 (20 µg/ml, 10 µg/ml, 5 µg/ml, 2.5 µg/ml, are incubated with the coated antigens for 1 hour at 37°C. After washing with PBS +Tween20 0.1% (SIGMA cod. PL379), plates are incubated with anti human IgG peroxidase (SIGMA cod. A2290) for 30 minutes at 37°C. After washing with PBS +Tween 0.1%, TMB substrate was added to the wells (PIERCE TMB substrate kit for peroxidase cod. SK 4400). ELISA plates are analysed with a spectrophotometer at 450nm. Results are shown in Fig.6.

### Example 15: Immunofluorescence with Fab 68 on human fibroblasts

Human Cytomegalovirus (HCMV) reference strain AD169 is propagated on MRC-5 fibroblasts (BioMérieux, Lyon, France). Confluent MRC-5 cells are conserved on Eagle's minimum essential medium (Invitrogen) containing 2% fetal calf serum (Sigma). For immunofluorescence staining human fibroblasts are seeded onto 24-well plates containing sterile glass coverslips. When cell cultures are confluent as monolayers, cells are infected as follows: medium is eliminated and cell monolayers are infected at high multiplicity of infection (about MOI 0,1) with HCMV. Plates are incubated at 37 ° C in the presence of 5% CO₂ for 96 hours. After incubation, media are removed and cells are fixed by 4% para-formaldehyde solution for 8 minutes. After washing with PBS solution cells are permeabilized with TritonX100 (0.1 % solution, SIGMA T-8787) and rinsed with PBS. After drying, cells are incubated with Fab68 and an unrelated negative control Fab for 1 hour at 37°C in a humid atmosphere. After washing with PBS, fixed cells are incubated with anti-human IgG Fab specific FITC-Conjugate (Sigma) for 30 minutes at 37°C in a humid atmosphere. The glass are mounted with glycerol buffer and observed by MRC 1024 Laser Scanning Confocal microscope (Biorad).

### Example 16: Synthesis of the peptides

### 12.1) General

Abbreviations for Chemical Reagents, Chemical Structure Moieties and Techniques: AA-amino acid, AcOH-Acetic acid, ACN-Acetonitrile, API-ES-Atmospheric pressure ionization electrospray, Btn-Biotin, Boc-tert-Butyloxycarbonyl, DCM- Dichloromethane, DIC-N,N-Diisopropylcarbodiimide, DIEA-N,N-Diisopropylethylamine, DMF- N,N-Dimethylformamide, Et₂O-Diethyl ether, Fmoc-9-Fluorenylmethoxycarbonyl, Adoa-8-Amino-3,6-dioxaoctanoic acid, HFIP-1,1,1,3,3,3-hexafluoro-2-propanol, HOBt-N-Hydroxybenzotriazole, MeOH-Methanol, Neg. ion-Negative ion, NHS-N-Hydroxysuccinimide, NMP-N-Methylpyrrolidone, Pip-Piperidine, Pos. ion-Positive ion, HBTU-O-(Benzotriazol-1-yl)-N,N,N',N'-tetramethyluronium hexafluorophosphate, PyBOP-Benzotriazole-1-yl-oxy-tris-pyrrolidino-phosphonium hexfluorophosphate, t_{R}-Retention time (minutes), Reagent B (88:5:5:2 - TFA:H₂O:phenol:TIPS - v/v/wt/v), Su-Succinimidyl, TFA-Trifluoroacetic Acid, TIPS-Triisopropylsilane, H₂O-Water.

Names, structures and abbreviations used for amines and unnatural amino acids used in the synthesis of various peptides are given in Table V.

Solvents for reactions, chromatographic purification and HPLC analyses are E. Merck Omni grade solvents from VWR Corporation (West Chester, PA). NMP and DMF are purchased from Pharmco Products Inc. (Brookfield, CT), and are peptide synthesis grade or low water/amine-free Biotech grade quality. Piperidine (sequencing grade, redistilled 99+%) and TFA (spectrophotometric grade or sequencing grade) are purchased from Sigma-Aldrich Corporation (Milwaukee, WI) or from the Fluka Chemical Division of Sigma-Alrich Corporation. Phenol (99%), DIEA, DIC and TIPS are purchased from Sigma-Aldrich Corporation. Fmoc-protected amino acids, PyBop, and HOBt used are purchased from Nova-Biochem (San Diego, CA, USA), Advanced ChemTech (Louisville, KY, USA), Chem-Impex International (Wood Dale III, USA), and Multiple Peptide Systems (San Diego, CA, USA). Fmoc-Adoa and Btn-Adoa-Adoa-OH are obtained from NeoMPS Corp (San Diego, CA).

Analytical HPLC data are generally obtained using a Shimadzu LC-10AT VP dual pump gradient system employing either Waters X-Terra® MS-C18 (5.0 p, 50 × 4.6 mm; 120Å pore size) or Waters Sunfire™ OBD-C8 (4.6 × 50 mm 3.5 p, 120Å pore size) columns and gradient or isocratic elution systems using H₂O (0.1% TFA) as eluent A and ACN (0.1% TFA) as eluent B. Detection of compounds is accomplished using UV at 220 and/or 230 nm.

Preparative HPLC is conducted on a Shimadzu LC-8A dual pump gradient system equipped with a SPD-10AV UV detector fitted with a preparative flow cell. Generally the solution containing the crude peptide is loaded onto a reversed phase Waters Sunfire™ OBD C8 (50 × 250 mm; particle size: 10.0 p, 120Å pore size) column, using a third pump attached to the preparative Shimadzu LC-8A dual pump gradient system. After the solution of the crude product mixture is applied to the preparative HPLC column the reaction solvents and solvents employed as diluents, such as DMF or DMSO, are eluted from the column at low organic phase composition. Then the desired product is eluted using a gradient elution of eluent B into eluent A. Product-containing fractions are combined based on their purity as determined by analytical HPLC and mass spectral analysis. The combined fractions are freeze-dried to provide the desired product.

Mass spectral data are obtained in-house on an Agilent LC-MSD 1100 Mass Spectrometer. For the purposes of fraction selection and characterization of the products, mass spectral values are usually obtained using API-ES in positive ion mode. Generally the molecular weight of the target peptides is -2000; the mass spectra usually exhibited strong doubly or triply positively charged ion mass values rather than weak [M+H]⁺. These are generally employed for selection of fractions for collection and combination to obtain the pure peptide from HPLC purification.

### 12.2) General Methods for Solid Phase Peptide Synthesis (SPPS)

12.2.1) The linear peptides are synthesized by an established automated protocol on a Rainin PTI Symphony^{®} Peptide Synthesizer (twelve peptide sequences/synthesis) using Fmoc-Pal-Peg-PS resin (0.2 mmol/g) and/or suitably preloaded resins, Fmoc-protected amino acids and PyBop-mediated ester activation in DMF. The rest of the peptide sequence is loaded on the Fmoc-Pal-Peg-PS and/or other resins in stepwise fashion by SPPS methods typically on a 0.2 mmol scale. The amino acid coupling is carried out with a 4-fold excess each of amino acid activated by PyBop-DIEA reagent in DMF. Biotin is coupled to N-terminus of the peptide with a four-fold excess of Btn-NHS ester.
12.2.2) When preloaded diamines on trityl resins are used, after final acetylation the fully protected peptide chain is cleaved from the resin with 8:1:1 - DCM: AcOH: HFIP and after evaporation of the volatiles, the final Btn-Adoa-Adoa is coupled to the amine at the C-terminus in solution. The crude fully protected peptide is treated with 1.0 equivalent of pre-formed Btn-Adoa-Adoa-NHS ester in solution for 16h at RT (total volume 5.0 mUg of the crude weight).

In a typical coupling process for a given amino acid, 6.0 mL of DMF solution containing 0.8 mmol of an amino acid followed by PyBOP (0.8 mmol, DMF solution, 1.25 mL) and DIEA (0.8 mmol, DMF solution, 1.25 mL) are added in succession by an automated protocol to a reaction vessel (RV) containing the resin (0.2 mmol) and the resin is agitated by recurrent nitrogen bubbling. After 1 hour the resin is washed thoroughly with DMF (4.5 mL, 6x) and the cleavage of the Fmoc-group is performed with 25% Pip in DMF (4.5 mL) for 10 minutes followed by a second treatment with the same reagent for 10 minutes to ensure complete deprotection. Again the resin is thoroughly washed with DMF (5 mUg, 6x); a DCM (10 mUg) wash is performed in between DMF washes to guarantee that the resin is freed of residual Pip. After completion of the peptide synthesis, the resin bearing the fully protected peptide was cleaved with, Reagent B (10.0 mUg of resin or 10.0 mUg of crude protected peptide) for 4 hours. The volatiles are removed under vacuum to provide a paste. The paste thus obtained is triturated with Et₂O to provide a solid which was pelleted by centrifugation followed by 3 more cycles of Et₂O washing and pelleting. The resulting solid is dried under vacuum to provide the crude peptide. A 200 µmol scale synthesis of a peptide of MW ∼ 2000 gave 200-300 mg (90-110% of theory) of the crude peptide. The greater than theoretical yield is most likely due to moisture and residual solvents.

### 12.3) Purification of peptides - General Procedure

A 200 µmol scale synthesis of a peptide of MW ∼ 2000 on the 'Symphony' instrument provided ∼ 200-300 mg of crude peptide from each reaction vessel (RV). The crude peptide (∼200-500 mg) is purified in one run by reversed phase HPLC. The crude peptide (-200 mg) dissolved in ACN (10 mL) is diluted to a final volume of 50 mL with H₂O and the solution is filtered. The filtered solution is loaded onto a preparative HPLC column (Waters, Sunfire™ Prep C8, 50 × 250 mm 10µ, 120Å) which had been pre-equilibrated with 10% ACN in H₂O (0.1% TFA). During the application of the solution to the column the flow of the equilibrating eluent from the preparative HPLC system is stopped. After the solution is applied to the column, the flow of equilibrating eluent from the gradient HPLC system is reinitiated and the composition of the eluent is then ramped to 20% ACN-H₂O (0.1%TFA) over 10.0 minutes after which a linear gradient at a rate of 0.5%/min of ACN (0.1% TFA) into H₂O (0.1% TFA) is initiated and maintained for 60 minutes. Fractions (15 mL) are collected using UV at 220 nm as an indicator of product elution. The collected fractions are analyzed on an analytical reversed phase C8 column (Waters Sunfire™ OBD-C8, 4.6 × 50 mm, 5µ, 120Å) and product-containing fractions of >95% purity are combined and freeze-dried to afford the corresponding peptide. After isolation, the peptides are analyzed by HPLC and mass spectrometry to confirm identity and purity. Data for the peptides are provided in Table VI (Sequence, Resin Loading and Yield), Table VII (HPLC and Mass Spectral Analysis) and Table VIII (Peptide Structures).

**Table V - Abbreviations and Structures**

| Abbreviation | Structure |
|---|---|
| Adoa | 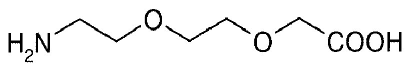 |
| Btn | 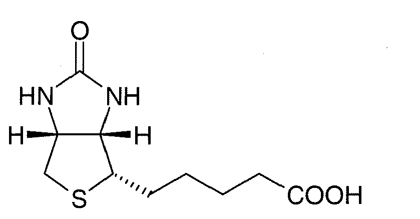 |
| EDA | H₂N-CH₂-CH₂-NH₂ |

**Table VI - Peptide Sequence, Resin Loading and Yield**

| CPD# | Sequence | Resin used, Loading, mmol/g, g, mmol | Yield in mg (%) |
|---|---|---|---|
| 1 | Ac-TMGFTAPRFPHY-NH₂ | Fmoc-PAL-PEG-PS, 0.2 mmol/g, 1.2 g, 0.24 mmol | 167.0 (46%) |
| 2 | Ac-MQSPFTPHFAER-NH₂ | Fmoc-PAL-PEG-PS, 0.2 mmol/g, 1.2 g, 0.24 mmol | 137.0 (38%) |
| 3 | Ac-MQSPIVLPLSLS-NH₂ | Fmoc-PAL-PEG-PS, 0.2 mmol/g, 1.2 g, 0.24 mmol | 131.0 (41%) |
| 4 | Ac-HHEPGAWLPLSP-NH₂ | Fmoc-PAL-PEG-PS, 0.2 mmol/g, 1.2 g, 0.24 mmol | 209.0 (62%) |
| 5 | Btn-Adoa-Adoa- TMGFTAPRFPHY-NH₂ | Fmoc-PAL-PEG-PS, 0.2 mmol/g, 1.0 g, 0.2 mmol | 70.0 (18%) |
| 6 | Btn-Adoa-Adoa-MQSPIVLPLSLS-NH₂ | Fmoc-PAL-PEG-PS, 0.2 mmol/g, 1.0 g, 0.2 mmol | 140.0 (38%) |
| 7 | Btn-Adoa-Adoa-HHEPGAWLPLSP-NH₂ | Fmoc-PAL-PEG-PS, 0.2 mmol/g, 1.0 g, 0.2 mmol | 205.0 (55%) |
| 8 | Btn-Adoa-Adoa-MQSPFTPHFAER-NH₂ | Fmoc-PAL-PEG-PS, 0.2 mmol/g, 1.0 g, 0.2 mmol | 135.0 (34%) |
| 9 | Ac-TMGFTAPRFPHY-DAE-Adoa-Adoa-Btn | 1,2-Diaminoethane trityl resin, 0.9 mmol/g, 0.222 g, 0.2 mmol | 65.0 (16%) |
| 10 | Ac-MQSPFTPHFAER-DAE-Adoa-Adoa-Btn | 12-Diaminoethane trityl resin, 0.9 mmol/g, 0.222 g, 0.2 mmol | 30.0 (7%) |
| 11 | Ac-MQSPIVLPLSLS-DAE-Adoa-Adoa-Btn | 1,2-Diaminoethane trityl resin, 0.9 mmol/g, 0.222 g, 0.2 mmol | 60.0 (15.7%) |
| 12 | Ac-HHEPGAWLPLSP-DAE-Adoa-Adoa-Btn | 1,2-Diaminoethane trityl resin, 0.9 mmol/g, 0.222 g, 0.2 mmol | 25.0 (7%) |

**Table VII-HPLC and Mass Spectral Analysis of Peptides**

| Cpd # | RT, Column & Conditions | MS |
|---|---|---|
| 1 | Ret. time: 7.38 min; Assay: >95% (area %); Column: Waters X-Terra MS C-18 RP, 50.0 mm × 4.6 mm i.d.; Particle size: 5.0 microns; Eluents: A: Water (0.1% TFA), B: acetonitrile (0.1% TFA); Elution: Initial condition: 10.0 % B, linear gradient 10-40% B over 15.0 min; Flow rate: 3.0 mL/min; Detection: UV @ 220 nm. | [M+H]: 1465.6, [M+2H]/2: 733.4 |
| 2 | Ret. time: 6.48 min; Assay: >95% (area %); Column: Waters X-Terra MS-C18 RP, 50.0 mm × 4.6 mm i.d.; Particle size: 5.0 microns; Eluents: A: Water (0.1% TFA), B: acetonitrile (0.1% TFA); Elution: Initial condition: 10.0% B, linear gradient 10-40% B over 15.0 min; Flow rate: 3.0 mL/min; Detection: UV @ 220 nm. | [M+H]: 1489.6; [M+Na+H]: 755.0; [M+2H]/2: 744.8 |
| 3 | Ret. time: 10.14 min; Assay: >95% (area %); Column: Waters X-Terra MS-C18 RP, 50.0 mm × 4.6 mm i.d.; Particle size: 5.0 microns; Eluents: A: Water (0.1% TFA), B: acetonitrile (0.1% TFA); Elution: Initial condition: 10.0% B, linear gradient 10-40 % B over 15.0 min; Flow rate: 3.0 mL/min; Detection: UV @ 220 nm. | [M+K]: 1364.6; [M+Na]: 1348.6 |
| 4 | Ret. time: 6.86 min; Assay: >95% (area %); Column: Waters X-Terra MS C-18 RP, 50.0 mm × 4.6 mm i.d.; Particle size: 5.0 microns; Eluents: A: Water (0.1% TFA), B: acetonitrile (0.1% TFA); Elution: Initial condition: 10.0% B, linear gradient 10-40% B over 15.0 min; Flow rate: 3.0 mL/min; Detection: UV @ 220 nm. | [M+H]: 1382.6; [M+Na]: 1403.6 |
| 5 | Ret. time: 5.63 min; Assay: >95% (area %); Column: Waters Sunfire™ C-8 RP, 50.0 mm × 4.6 mm i.d.; Particle size: 3.5 microns; Eluents: A: Water (0.1% TFA), B: acetonitrile (0.1% TFA); Elution: Initial condition: 15.0% B, linear gradient 15-45% B over 15.0 min; Flow rate: 3.0 mL/min; Detection: UV @ 220 nm. | [M+H]: 1940.6; [M+2H]/2: 970.8 |
| | | |
| 6 | Ret. time: 8.53 min; Assay: >95% (area %); Column: Waters Sunfire™ C-8 RP, 50.0 mm × 4.6 mm i.d.; Particle size: 3.5 microns; Eluents: A: Water (0.1% TFA), B: acetonitrile (0.1% TFA); Elution: Initial condition: 15.0% B, linear gradient 15-45% B over 15.0 min; Flow rate: 3.0 mL/min; Detection: UV @ 220 nm | [M+Na]: 1823.8; [M+H]: 1800.8; [M+2Na]/2; 922.5; [M+2H]/2: 900.5 |
| 7 | Ret. time: 5.57 min; Assay: >95% (area %); Column: Waters Sunfire™ C-8 RP, 50.0 mm × 4.6 mm i.d.; Particle size: 3.5 microns; Eluents: A: Water (0.1% TFA), B: acetonitrile (0.1% TFA); Elution: Initial condition: 15.0% B, linear gradient 15-45% B over 15.0 min; Flow rate: 3.0 mL/min; Detection: UV @ 220 nm | [M+H]: 1855.5; [M+2H]/2: 928.5 |
| 8 | Ret. time: 5.29 min; Assay: >95% (area %); Column: Waters Sunfire™ C-8 RP, 50.0 mm × 4.6 mm i.d.; Particle size: 3.5 microns; Eluents: A: Water (0.1% TFA), B: acetonitrile (0.1% TFA); Elution: Initial condition: 15.0% B, linear gradient 15-45% B over 15.0 min; Flow rate: 3.0 mL/min; Detection: UV @ 220 nm | [M+H]: 1964.5; [M+2H]/2: 982.0 |
| 9 | Ret. time: 5.73 min; Assay: >95% (area %); Column: Waters Sunfire™ C-8 RP, 50.0 mm × 4.6 mm i.d.; Particle size: 3.5 microns; Eluents: A: Water (0.1% TFA), B: acetonitrile (0.1% TFA); Elution: Initial condition: 15.0% B, linear gradient 15-45% B over 15.0 min; Flow rate: 3.0 mL/min; Detection: UV @ 220 nm | [M+H]: 2025.5; [M+2H]/2: 1013.3 |
| 10 | Ret. time: 5.29 min; Assay: >90% (area %); Column: Waters Sunfire™ C-8 RP, 50.0 mm × 4.6 mm i.d.; Particle size: 3.5 microns; Eluents: A: Water (0.1% TFA), B: acetonitrile (0.1% TFA); Elution: Initial condition: 15.0% B, linear gradient 15-45% B over 15.0 min; Flow rate: 3.0 mL/min; Detection: UV @ 220 nm | [M+H]: 2047.8; [M+2H]/2: 1024.7 |
| 11 | Ret. time: 8.41 min; Assay: >90% (area %); Column: Waters Sunfire™ C-8 RP, 50.0 mm × 4.6 mm i.d.; Particle size: 3.5 microns; Eluents: A: Water (0.1% TFA), B: acetonitrile (0.1% TFA); Elution: Initial condition: 15.0% B, linear gradient 15-45% B over 15.0 min; Flow rate: 3.0 mL/min; Detection: UV @ 220 nm | [M+H]: 1906.8; [M+2H]/2: 965.0 |
| 12 | Ret. time: 5.73 min; Assay: >95% (area %); Column: Waters Sunfire™ C-8 RP, 50.0 mm × 4.6 mm i.d.; Particle size: 3.5 microns; Eluents: A: Water (0.1% TFA), B: acetonitrile (0.1% TFA); Elution: Initial condition: 15.0% B, linear gradient 15-45% B over 15.0 min; Flow rate: 3.0 mL/min; Detection: UV @ 220 nm | [M+H]: 1940.8; [M+2H]/2: 971.0 |

**Table VIII - Structures of peptides**

| **Compound 1** **Ac-TMGFTAPRFPHY-NH₂** |
|---|
| 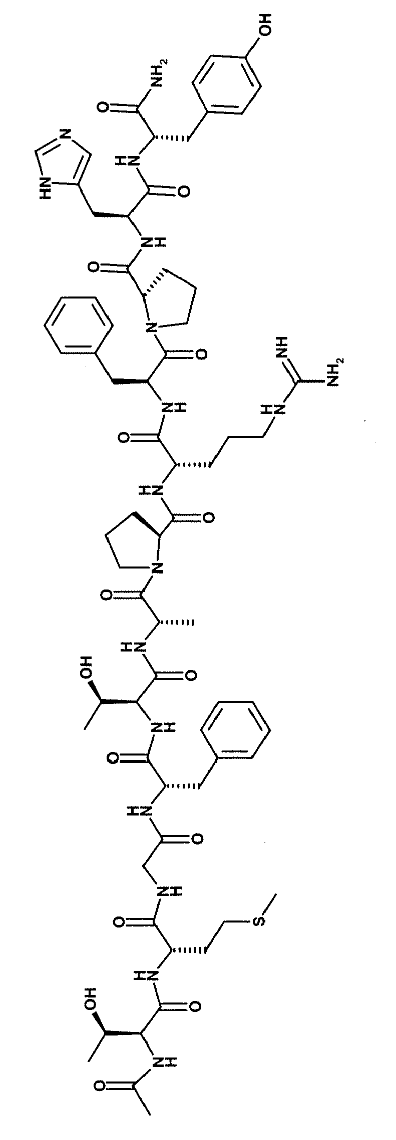 |

| **Compound 2** **Ac-MQSPFTPHFAER-NH₂** |
|---|
| 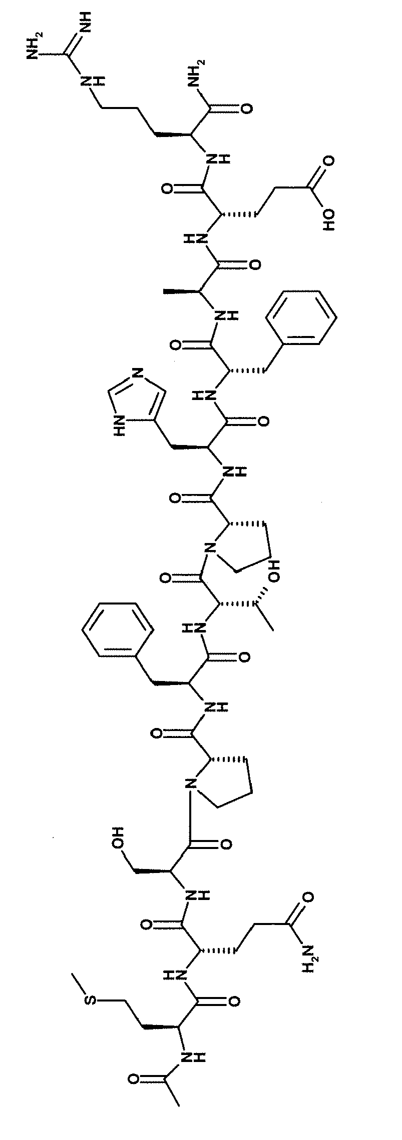 |

| **Compound 3** **Ac-MOSPIVLPLSLS-NH₂** |
|---|
| 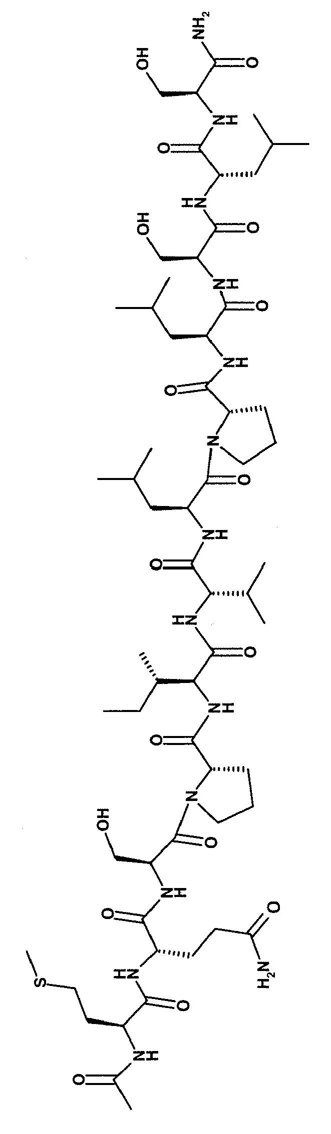 |

| **Compound 4** **Ac-HHEPGAWLPLSP-NH₂** |
|---|
| 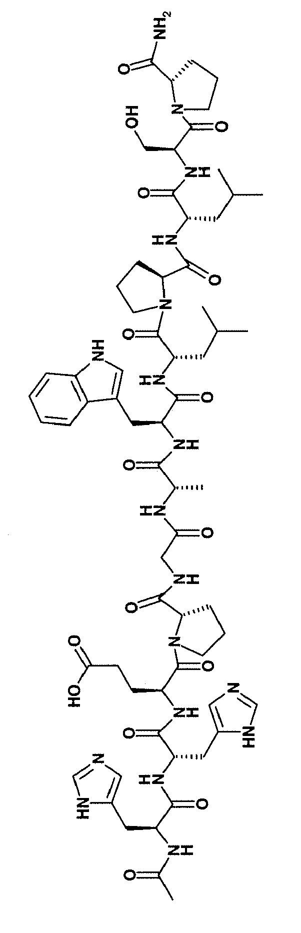 |

| **Compound 5** **Btn-Adoa-Adoa-TMG FTAPRFPHY-NH₂** |
|---|
| 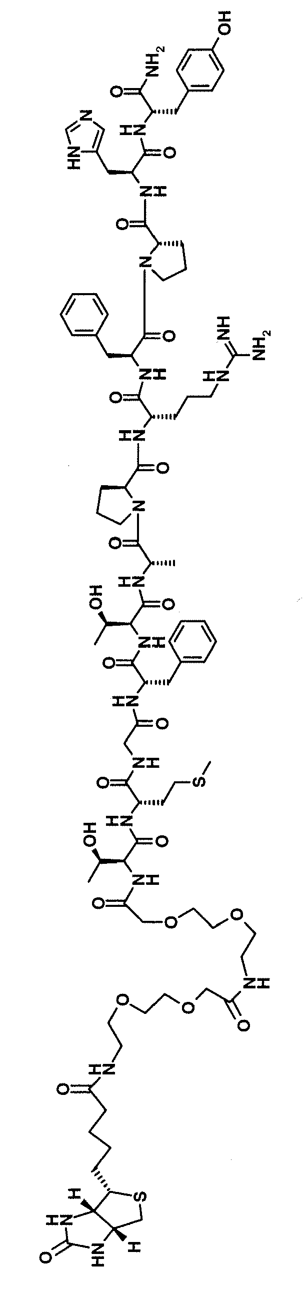 |

| **Compound 6** **Btn-Adoa-Adoa-MOSPIVLPLSLS-NH₂** |
|---|
| 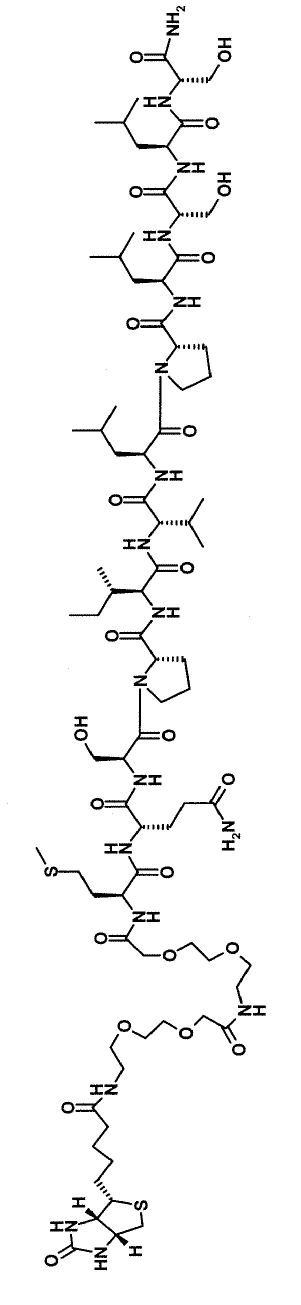 |

| **Compound 7** **Btn-Adoa-Adoa-HHEPGAWLPLSP-NH₂** |
|---|
| 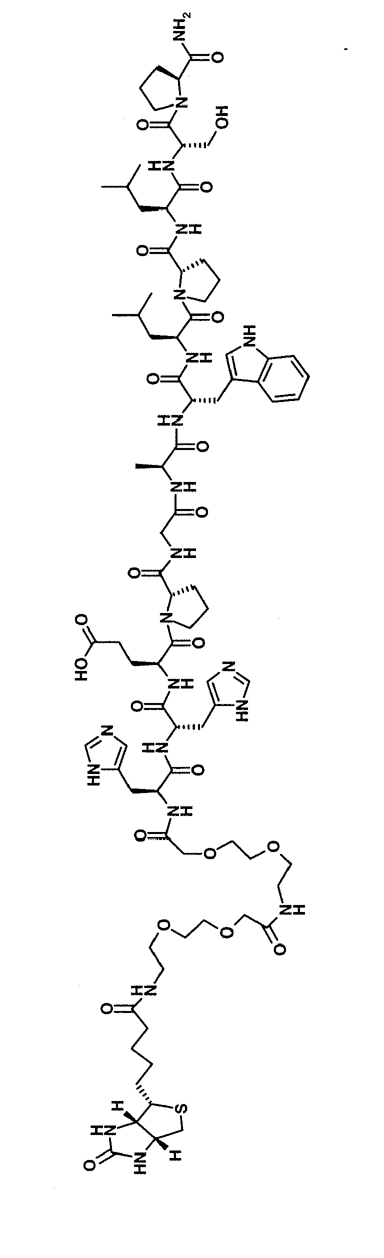 |

| **Compound 8** **Btn-Adoa-Adoa-MQSPFTPHFAER-NH₂** |
|---|
| 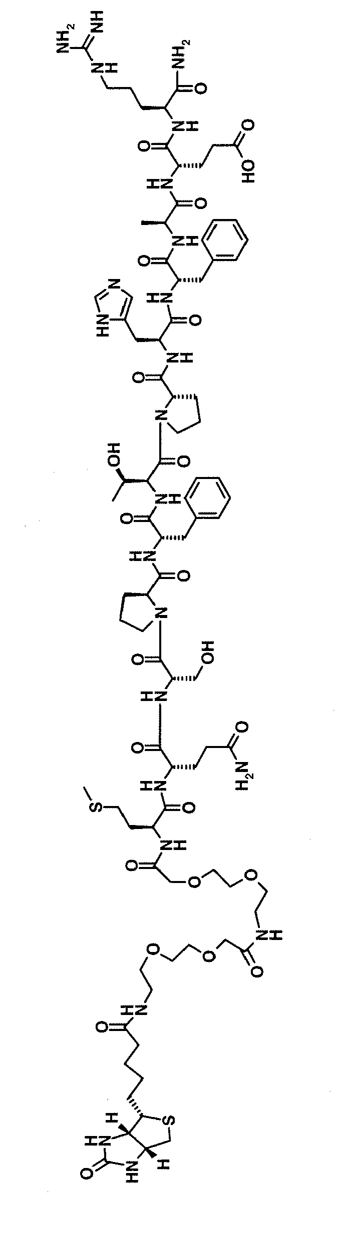 |

| **Compound 9** **Ac-TMGFTAPRFPHY-DAE-Adoa-Adoa-Btn** |
|---|
| 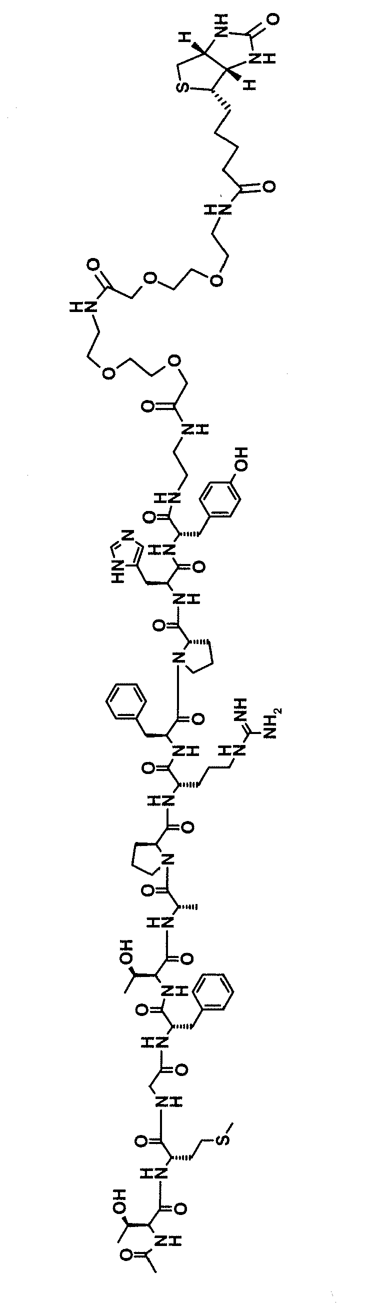 |

| **Compound 10** **Ac- MQSPFTPHFAER-DAE-Adoa-Adoa-Btn** |
|---|
| 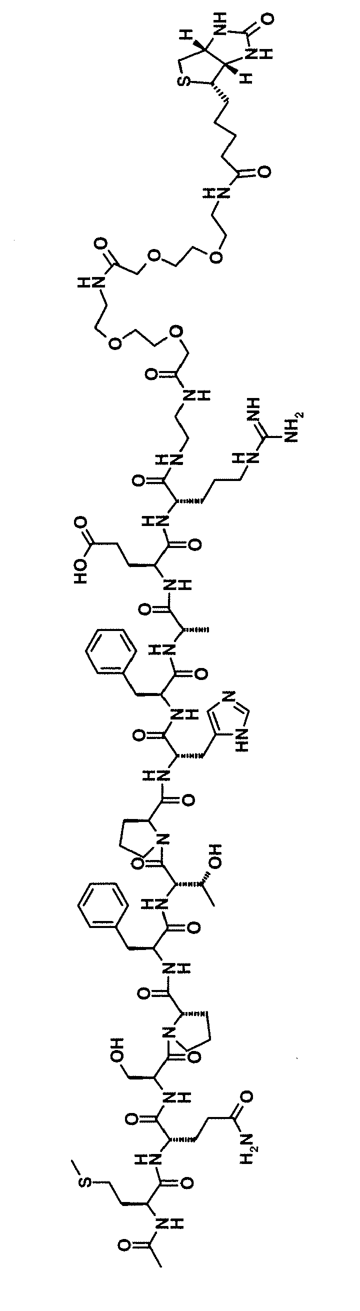 |

| **Compound 11** **Ac-MQSPIVLPLSLS-DAE-Adoa-Adoa-Btn** |
|---|
| 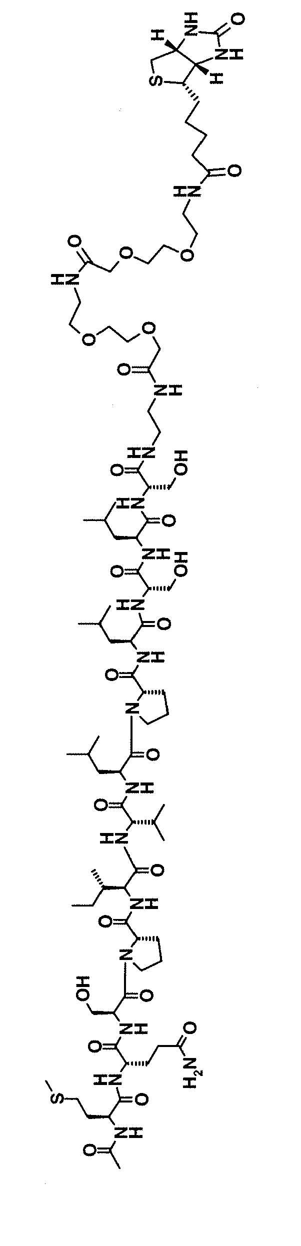 |

| **Compound 12** **Ac- HHEPGAWLPLSP-DAE-Adoa-Adoa-Btn** |
|---|
| 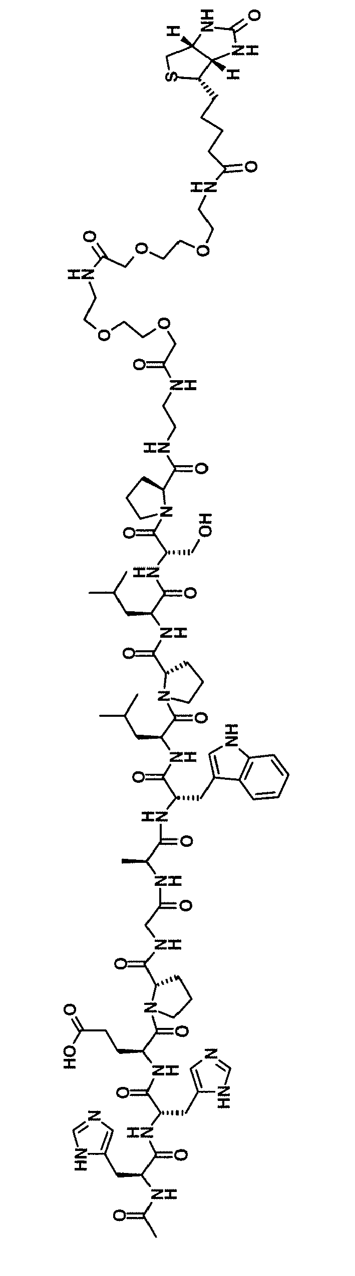 |

SEQUENCE LISTING

Sequence ID 1

ctcgaggagt ctggagcaga agtgaagaag ccgggcgaaa atcttaagat ctcctgcgag 60

gcttctggat acaattttgt caatcactgg atcggctggg tgcgccagat gcccgggaga 120

ggccttgagt ggatgggccg catctatcct ggagactctg aaaccagatt cagtccgtcc 180

ttccaagggc aggtcaccat ctcagtcgac aaaactctga gtaccgcctc cctacagtgg 240

aacagtctca agacgtcgga cagcgccaca tattattgtg tgacactggg gcgctggagc 300

agctggcaag gtggggcgct ctcatggggc cagggaacc 339

Sequence ID 2

Leu Glu Glu Ser Gly Ala Glu Val Lys Lys Pro Gly Glu Asn Leu Lys
1               5                   10                  15

Ile Ser Cys Glu Ala Ser Gly Tyr Asn Phe Val Asn His Trp Ile Gly
            20                  25                  30

Trp Val Arg Gln Met Pro Gly Arg Gly Leu Glu Trp Met Gly Arg Ile
        35                  40                  45

Tyr Pro Gly Asp Ser Glu Thr Arg Phe Ser Pro Ser Phe Gln Gly Gln
    50                  55                  60

Val Thr Ile Ser Val Asp Lys Thr Leu Ser Thr Ala Ser Leu Gln Trp
65                  70                  75                  80

Asn Ser Leu Lys Thr Ser Asp Ser Ala Thr Tyr Tyr Cys Val Thr Leu
                85                  90                  95

Gly Arg Trp Ser Ser Trp Gln Gly Gly Ala Leu Ser Trp Gly Gln Gly
            100                 105                 110

Thr

Sequence ID 3

gagctcacgc agtctccagc caccgtgtct gtgtctccag gggaaagagc caccctctcc 60

tgcagggcca gtcagagtat tagtttccac ttagcctggt accagcagaa acctggccag 120

gctcccagtc tcctcatcta cggaacatcc accagggcca ctggtatccc agccaggttc 180

agtggcagtg ggtctgggac agagttcact ctcaccatca gcagcctgca gtctgaagat 240

tctgcggttt attactgtca gcagtatcat aactggcctc ccctcacttt cggcggaggg 300

acc 303

Sequence ID 4

Glu Leu Thr Gln Ser Pro Ala Thr Val Ser Val Ser Pro Gly Glu Arg
1               5                   10                  15

Ala Thr Leu Ser Cys Arg Ala Ser Gln Ser Ile Ser Phe His Leu Ala
            20                  25                  30

Trp Tyr Gln Gln Lys Pro Gly Gln Ala Pro Ser Leu Leu Ile Tyr Gly
        35                  40                  45

Thr Ser Thr Arg Ala Thr Gly Ile Pro Ala Arg Phe Ser Gly Ser Gly
    50                  55                  60

Ser Gly Thr Glu Phe Thr Leu Thr Ile Ser Ser Leu Gln Ser Glu Asp
65                  70                  75                  80

Ser Ala Val Tyr Tyr Cys Gln Gln Tyr His Asn Trp Pro Pro Leu Thr
                85                  90                  95

Phe Gly Gly Gly Thr
            100

Sequence ID 5

Thr Met Gly Phe Thr Ala Pro Arg Phe Pro His Tyr
1               5                   10

Sequence ID 6

Met Gln Ser Pro Phe Thr Pro His Phe Ala Glu Arg
1               5                   10

Sequence ID 7

Met Gln Ser Pro Ile Val Leu Pro Leu Ser Leu Ser
1               5                   10

Sequence ID 8

His His Glu Pro Gly Ala Trp Leu Pro Leu Ser Pro
1               5                   10

## Claims

1. A process for the preparation of recombinant antibodies or of any fragments thereof including the steps of:
a) preparing an expression system comprising a host cell comprising
- one expression vector comprising two polynucleotidic molecules corresponding to Sequences ID 1 and 3; or
- two expression vectors each comprising one of the two polynucleotidic molecules corresponding to Sequences ID 1 and 3;
b) culturing said host cell under suitable growth conditions;
c) recovering and purifying the antibodies or any fragments thereof thus produced.

2. The process of claim 1 wherein the host cell is selected from the group consisting of prokaryotic, yeast and eukaryotic cells.

3. The process according to claim 2 wherein prokaryotic cells are selected among *Enterobacter, Escherichia, Etwinia, Klebsiella, Proteus, Salmonella, Serratia, Shigella, Bacilli, Pseudomonas* and *Streptomyces.*

4. The process according to claim 3 wherein prokaryotic cells are selected among *E. coli, Salmonella typhimurium, Serratia marcescans, Bacillus subtilis, Bacillus licheniformis, Pseudomonas aeruginosa.*

5. The process according to claim 2 wherein yeast cells are selected among *Saccharomyces, Pichia pastoris, Kluyveromyces* such as *K*. *lactis, K. fragilis, K. bulgaricus, K. wickeramii, K. waltii, K. drosophilarum, K. thermotolerans, K. marxianus, Schizosaccharomyces,* such as *Schizosaccharomyces pombe, yarrowia, Hansenula, Trichoderma reesia, Neurospora crassa, Schwanniomyces* such as *Schwanniomyces occidentalis, Neurospora, Penicillium, Tolypociadium, Aspergillus* such as A. *nidulans, Candida, Torulopsis* and *Rhodotorula.*

6. The process according to claim 2 wherein eukaryotic cells are selected among Chinese hamster ovary (CHO), monkey kidney CVI line transformed by SV40 (COS-7, ATCC CRL 1651), human embryonic kidney line, Chinese hamster ovary cells/-DHFR, mouse sertoli cells, human lung cells (W138, ATCC CCL 75); human liver cells (Hep G2, HB 8065); and mouse mammary tumor (MMT 060562, ATCC CCL51); plant isolated recombinant host cells such as *Agrobacterium tumefaciens* and *Nicotiana tabacum*; insect recombinant isolated cells such as *Drosophila* S2 and *Spodoptera Sf9.*

7. A process according to claim 1 wherein the expression vector is selected from the group consisting of plasmids, cosmids, YACs, viral particles or phages.

8. A process according to anyone of the claims from 1 to 7 for the preparation of IgG antibodies or fragments thereof and IgG antibodies Fab fragments.

9. A recombinant antibody selected from IgG antibodies or any fragment thereof and IgG Fab fragments obtainable according to the process of anyone of the claims from 1 to 7.

10. A recombinant antibody selected from IgG antibodies or any fragment thereof and IgG Fab fragments comprising the amino acidic sequences ID 2 and 4.

11. A recombinant antibody according to claim 10 wherein the heavy chain corresponds to Sequence ID 2 and light chain corresponds to Sequence ID 4.

12. The recombinant antibody according to anyone of claims from 9 to 11 which bind to the antigen possibly present in the coronary plaque.

13. A therapeutic composition comprising a recombinant antibody or any fragment thereof or Fab fragments according to anyone of claims from 9 to 12 and, optionally, a therapeutic moiety.

14. The composition of claim 13 wherein the therapeutic moiety is selected from the group consisting of radionuclides, drugs and prodrugs, hormones, hormone antagonists, receptor antagonists, enzymes or proenzymes activated by another agent, autocrines or cytokines, antimicrobial agents and toxins.

15. The composition of claim 13 or 14 for the treatment of the acute coronary syndrome (ACS).

16. A diagnostic composition comprising a recombinant antibody or any fragment thereof or Fab fragments according to anyone of claims from 9 to 12 and a diagnostic moiety.

17. The composition of claim 16 for the diagnosis of the acute coronary syndrome (ACS).

18. A ligand which binds to the recombinant antibody or to any fragment thereof or Fab fragments according to anyone of claims from 9 to 12.

19. The ligand of claim 18 which is selected by a method including the use of the recombinant antibody according to anyone of claims from 9 to 12.

20. A method for the identification of a ligand which binds to the recombinant antibodies or to any fragment thereof or Fab fragments according to anyone of claims from 9 to 12, said method including the steps of:
a) binding said antibodies or any fragment thereof onto a solid phase;
b) removing unbound material by one or more washing steps;
c) contacting the candidate ligand with the solid phase prepared in step a) and allowing incubation of the candidate ligand and the solid phase for a suitable period of time;
d) removing unbound material by one or more washing steps;
e) adding a secondary antibody specific for the complex of the antibody of step a) with the candidate ligand bound thereto; and
f) identifying the bound ligand to the antibodies of step a).

21. An *ex-vivo* or *in vitro* diagnostic method comprising the step of contacting a sample selected from whole blood, serum and coronary plaque fragment with the antibody or any fragment thereof or Fab fragments according to anyone of claims from 9 to 12.

22. The *ex-vivo* or *in vitro* diagnostic method of claim 21 for the diagnosis of acute coronary syndrome (ACS) in a patient.

23. The *ex-vivo* or *in vitro* diagnostic method of claim 21 for the screening of the population at risk of acute coronary syndrome (ACS).
